(19) Europäisches Patentamt
European Patent Office
Office européen des brevets

(11) Numéro de publication : **0 636 609 A1**

# (12) DEMANDE DE BREVET EUROPEEN

(21) Numéro de dépôt : **94401738.3**

(22) Date de dépôt : **28.07.94**

(51) Int. Cl.[6] : **C07D 209/96,** C07D 471/10, C07D 491/10, // (C07D471/10, 221:00, 209:00), (C07D491/10, 311:00, 209:00)

(30) Priorité : **30.07.93 FR 9309405**

(43) Date de publication de la demande : **01.02.95 Bulletin 95/05**

(84) Etats contractants désignés : **AT BE CH DE DK ES FR GB GR IE IT LI LU NL PT SE**

(71) Demandeur : **SANOFI**
**32-34, rue Marbeuf**
**F-75008 Paris (FR)**

(72) Inventeur : **Foulon, Loic**
**14, rue de l'Ousse**
**F-31120 Pinsaguel (FR)**
Inventeur : **Garcia, Georges**
**27 rue des Aires**
**F-34980 Saint Gely Du Fesc (FR)**
Inventeur : **Mettefeu, Daniel**
**13 rue du Bosquet**
**F-34790 Grabels (FR)**
Inventeur : **Serradeil-Legal, Claudine**
**45 Avenue des Troubadours**
**F-31750 Escalquens (FR)**
Inventeur : **Valette, Gérard**
**8 rue de Montségur**
**F-31120 Lacroix-Sasgarde (FR)**

(74) Mandataire : **Gillard, Marie-Louise**
**Cabinet Beau de Loménie**
**158, rue de l'Université**
**F-75340 Paris Cédex 07 (FR)**

(54) **Dérivés du 1-benzyl-1,3-dihydro-indol-2-one, leur préparation, les compositions pharmaceutiques en contenant.**

(57) L'invention concerne des dérivés du 1-benzyl-1,3-dihydro-indol-2-one de formule :

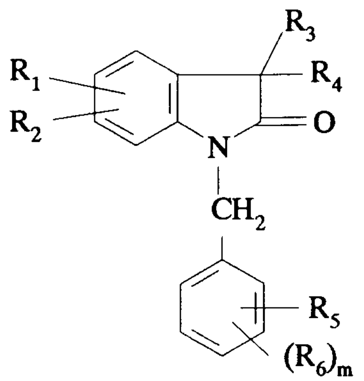

(I)

et leurs sels éventuels, ainsi que leur préparation et les compositions pharmaceutiques en contenant.
Ces composés ont une affinité pour les récepteurs de la vasopressine et/ou de l'ocytocine.

EP 0 636 609 A1

La présente invention a pour objet des dérivés du 1-benzyl-1,3-dihydro-indol-2-one, leur préparation et les compositions pharmaceutiques en contenant.

Plusieurs brevets ou demandes de brevets décrivent des dérivés 1-benzyl-1,3-dihydro-indol-2-ones.

La demande de brevet JP-87/227987 décrit la préparation de dérivé d'oxothiolane de formule :

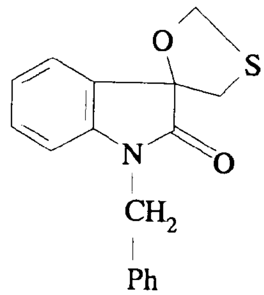

1

Les demandes de brevets EP 28906 et EP 66378 décrivent des composés de formule :

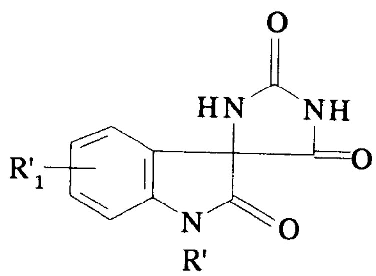

2

dans laquelle R' peut représenter un groupement $CH_2Ph$, plus particulièrement $3,4\text{-}Cl_2C_6H_3CH_2$. Ces composés diminuent le niveau de sorbitol dans le nerf sciatique.

Le brevet US 4226860 décrit des composés de formule :

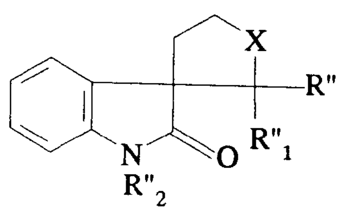

3

dans laquelle :

- R" est un alkyle ou un carboxyalkyle,
- $R"_1$ est un alkyle,
- $R"_2$ est un alkyle ou phénylalkyle,
- X est un méthylène ou un oxygène.

Ces composés sont utiles pour le traitement de l'hypertension.

Le brevet DE 3529994 décrit des composés de formule :

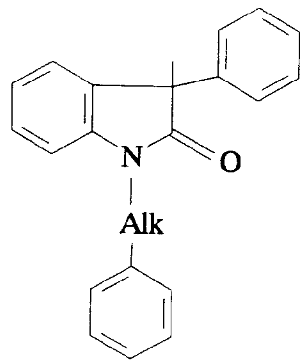

4

dans laquelle chacun des 3 noyaux phényles peut porter divers substituants. Ces composés sont des antagonistes calciques.

La demande de brevet EP 344634 décrit des dérivés indolones ou quinolones de formule :

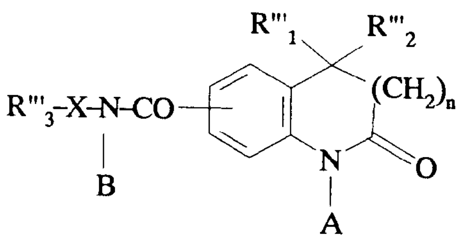

dans laquelle :

- n = 0 ou 1
- A et B représentent l'hydrogène, un alkyle, un alcényle, un alcynyle, un benzyle ou un cycloalkyle,
- R'''$_1$ et R'''$_2$ représentent indépendamment un alkyle, un alcényle ou un cycloalkyle ou R'''$_1$ et R'''$_2$ représentent ensemble un cycloalcane en $C_3$-$C_7$.

Ces composés sont des inhibiteurs de l'agrégation plaquettaire.

Le brevet US 4806651 décrit la préparation de composés de formule :

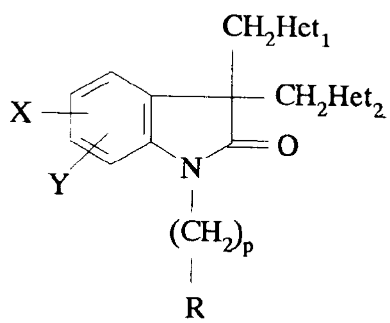

5

dans laquelle :

- $Het_1$ et $Het_2$ représentent des hétérocycles,
- R peut représenter un phényle substitué,
- p est zéro ou un.

Ces composés sont utiles pour traiter les dysfonctionnements cognitifs ou neurologiques.

Récemment, plusieurs demandes de brevet ont décrit des familles de composés à structure non-peptidique ayant une activité sur les récepteurs de la vasopressine et/ou de l'ocytocine. On peut citer les demandes EP 382 185, EP 444 945 et EP 514 667, EP 469 984, EP 526 348 et EP 581 939, les demandes WO 91/05 549, WO 93/15051, et plus particulièrement la demande de brevet JP-03/127732. Cette dernière décrit des dérivés de l'acide indole-3-propanoique de formule :

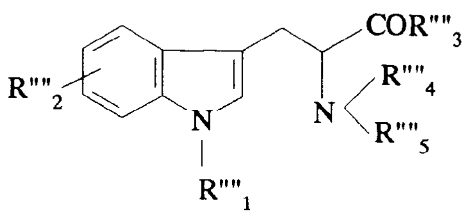

6

dans laquelle

- $R''''_1$ représente l'hydrogène, un alkyle, un alcényle, un phénylalkyle, un tétrahydrofuryle, un alcoxycarbonyle, un alcoxycarbonylalkyle, un carboxyalkyle ou un alcanoyle ;
- $R''''_2$ représente l'hydrogène, un hydroxyle, un alcoxy, un alkyle, un phénylalkyle, un phénylalcoxy ou un halogène ;
- $R''''_3$ représente un hydrogène, un alcoxy, un groupe amino libre ou substitué ou un résidu d'aminoacide ;
- $R''''_4$ représente l'hydrogène, un alkyle ou un phénylalkyle;
- $R''''_5$ représente un benzoyle, un phényle, un alkyle, un phénylalcénylcarbonyle, un thiénylcarbonyle, un phénylsulfonyle, un pyridylcarbonyle ou un imidazolylcarbonyle, les groupes phényle et alkyle du substituant $R''''_5$ pouvant être substitués.

Ces composés sont des antagonistes de la vasopressine.

Le brevet US 4 803 217 revendique des hapalindolinones obtenues par fermentation qui sont des antagonistes de la vasopressine. Ces composés sont représentés par la formule suivante :

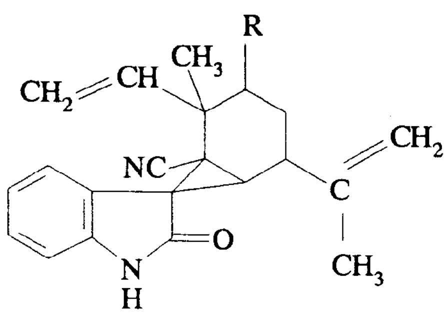

7

dans laquelle R représente H ou Cl.

Les dérivés du 1-benzyl-1,3-dihydro-indol-2-one selon la présente invention ont une affinité pour les récepteurs de la vasopressine et de l'ocytocine.

La vasopressine est une hormone connue pour son effet antidiurétique et son effet dans la régulation de la pression artérielle. Elle stimule plusieurs types de récepteurs : $V_1$ ($V_{1a}$, $V_{1b}$), $V_2$. Ces récepteurs sont localisés dans le foie, les vaisseaux (coronaires, rénaux, cérébraux), les plaquettes, le rein, l'utérus, les glandes surrénales, le système nerveux central, l'hypophyse. L'ocytocine a une structure peptidique proche de celle de la vasopressine. Les récepteurs de l'ocytocine se trouvent aussi sur le muscle lisse de l'utérus, ils se trouvent également sur des cellules myoépithéliales de la glande mammaire, dans le système nerveux central et dans le rein. La localisation des différents récepteurs est décrite dans : S. JARS et al., Vasopressin and oxytocin receptors : an overview, *in* Progress in Endocrinology. H. IMURA and K. SHIZURNE ed., Experta Medica, Amsterdam, 1988, 1183-1188, ainsi que dans les articles suivants. Presse Médicale, 1987, 16 (10), 481-485, J. Lab. Clin. Med., 1989, 114 (6), 617-632 et Pharmacol. Rev., 1991, 43 (1), 73-108. La vasopressine exerce ainsi des effets cardiovasculaires, hépatiques, antidiurétiques, agrégants et des effets sur le système nerveux central et périphérique et sur la sphère utérine. L'ocytocine intervient dans la parturition, la lactation et le comportement sexuel.

Les composés selon la présente invention permettent soit de mimer les effets de l'hormone (pour les agonistes), soit de les inhiber (pour les antagonistes), de façon sélective. Les antagonistes des récepteurs de la vasopressine peuvent intervenir sur la régulation de la circulation centrale et périphérique, notamment les circulations coronaire, rénale et gastrique, ainsi que sur la régulation hydrique et la libération de l'hormone adrénocorticotrophique (ACTH). Les agonistes de la vasopressine peuvent remplacer avantageusement la vasopressine ou ses analogues dans le traitement du diabète insipide ; ils peuvent également être utilisés dans le traitement de l'énurésie, et dans la régulation de l'hémostase : traitement de l'hémophilie, du syndrome de Von Willebrand, antidote des agrégants plaquettaires, F.A. LASZLO, , Pharmacol. Rev., 1991, 43, 73-108. Drug

Investigation, 1990, 2 (Suppl. 5), 1-47. Les hormones elles-mêmes : la vasopressine et l'ocytocine ainsi que certains de leurs analogues peptidiques ou non peptidiques sont utilisés en thérapeutique et ont montré leur efficacité. On peut citer plusieurs revues et de nombreux articles de la littérature Vasopressin. P. GROSS et al. ed. John Libbey Eurotext, 1993, en particulier 243-257 et 549-562. F.A. LASZLO and F.A. LASZLO Jr., Clinical perspectives for vasopressin antagonists, Drug News Perspect., 1993, 6 (8) ; W.G. NORTH, J. Clin. Endocrinol., 1991, 73, 1316-1320. J.J. LEGROS et al., Prog. Neuro-Pharmacol. Biol. Psychiat., 1988, 12, 571-586 ; K.E. ANDERSSON et al., Drugs Today, 1988, 24 (7) 509-528 ; D.L. STUMP et al., Drugs, 1990, 39, 38-53 ; S. CALTABIANO et al., Drugs Future, 1988, 13, 25-30 ; Y. MURA et al., Clin. Nephrol. 1993, 40, 60-61 ; FASEB J., 1994, 8(5), A587 ; 3398.

Ainsi les composés selon l'invention sont utiles notamment dans le traitement des affections du système nerveux central et périphérique, du système cardiovasculaire, de la sphère rénale, de la sphère gastrique et dans les troubles du comportement sexuel, chez l'homme et chez l'animal.

La présente invention a pour objet des composés de formule :

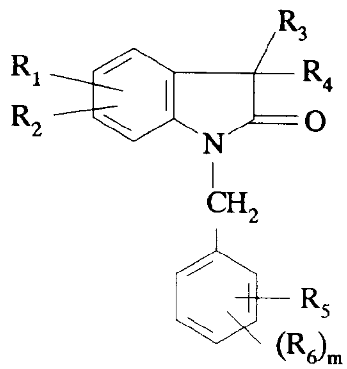

(I)

dans laquelle

- $R_1$ et $R_2$ représentent chacun indépendamment un hydrogène ; un halogéno ; un hydroxy ; un ω-halogéno($C_1$-$C_7$)alcoxy ; un ($C_1$-$C_7$)alkyle ; un trifluorométhyle ; un ($C_1$-$C_7$)alcoxy ; un polyhalogéno($C_1$-$C_7$)alcoxy ; un ω-hydroxy($C_2$-$C_7$)alcoxy, un ω-méthoxy($C_2$-$C_7$)alcoxy ; un ω-amino($C_2$-$C_7$)alcoxy libre ou substitué par un ou deux ($C_1$-$C_7$)alkyles ; un ($C_3$-$C_7$)cycloalkyloxy ; un ($C_3$-$C_7$)cycloalkylméthoxy ; un phénoxy ; un benzyloxy ; un ($C_1$-$C_7$)alkylthio ; un phénylthio ; un nitro ; un amino libre ou substitué par un ou deux ($C_1$-$C_7$)alkyles ; un cyano ; un formyle ; un ($C_1$-$C_7$)alkylcarbonyle ; un benzoyle ; un formyloxy ; un ($C_1$-$C_7$)alkylcarbonyloxy ; un benzoyloxy ; un ($C_1$-$C_7$)alkylsulfonamido ; un phénylsulfonamido ; un benzylsulfonamido ; un ($C_1$-$C_7$)alkylcarbonylamino ; un ($C_1$-$C_7$)alcoxycarbonylamino ; un uréido non substitué ou substitué par un phényle, un benzyle ou par un ou deux ($C_1$-$C_7$)alkyles ; un thiouréido non substitué ou substitué par un phényle, un benzyle ou par un ou deux ($C_1$-$C_7$)alkyles ; à la condition que $R_1$ et $R_2$ ne soient pas simultanément l'hydrogène ;
- $R_3$ et $R_4$ ensemble constituent un groupe $-(CH_2)_pX(CH_2)_q-$ ;
  ou
- $R_3$ et $R_4$ ensemble avec le carbone auquel ils sont liés constituent un cycle hydrocarboné en $C_3$-$C_{12}$, saturé ou insaturé, éventuellement condensé, non substitué ou substitué par un ou plusieurs groupes ($C_1$-$C_7$)alkyles, par un spirocycloalkyle en $C_3$-$C_5$, par un groupe oxo, par un ou deux hydroxy libres ou substitués par un groupe choisi parmi les groupes : ($C_1$-$C_4$)alkyle, ($C_1$-$C_2$)alcoxy($C_1$-$C_4$)alkyle, phényl($C_1$-$C_2$)alcoxy($C_1$-$C_4$)alkyle, tétrahydrofuranyle et tétrahydropyranyle ;
- $R_5$ représente l'hydrogène ou l'une des valeurs désignées pour $R_5$ ;
- $R_5$ représente un halogène ; un ($C_1$-$C_7$)alkyle ; un trifluorométhyle ; un cyano ; un nitro ; un hydroxylamino ; un hydroxy ; un carboxy ; un guanidino non substitué ou substitué en position 1 par un ($C_1$-$C_7$)alkyle et/ou en position 3 par un ou deux ($C_1$-$C_7$)alkyles, un phényle ou un benzyle et/ou en position 2 par un cyano ; un groupe $OR_7$ ; un groupe $SR_7$ ; un formyle ; un ($C_1$-$C_7$)alkylcarbonyle ; un benzoyle ; un ($C_1$-$C_7$)alcoxycarbonyle ; un phénoxycarbonyle ; un benzyloxycarbonyle ; un groupe $-CONR_{17}R_{18}$ ; un groupe $-CSNR_{11}R_{19}$ ;un groupe $-SO_2NR_{20}R_{21}$ ; un ($C_1$-$C_7$)alkylsulfonamido ; un groupe $-NHSO_2$-Ar ; un benzylsulfonamido ; un aminosulfonamido dans lequel l'amino est libre ou substitué par $R_{16}$ et $R_{22}$ ; un groupe $-NR_5R_9$ ; un groupe $-CO-NH-CR_{10}R_{23}-COR_{12}$ ; un groupe $-CH_2NR_5R_9$ ;
- $R_7$ représente un ($C_1$-$C_7$)alkyle ; un phényle ; un benzyle ; un ($C_3$-$C_7$)cycloalkyle ; un ($C_2$-$C_7$)alcényle ; un ω-halogéno($C_2$-$C_7$)alkyle ; un polyhalogéno($C_1$-$C_7$)alkyle ; un ω-hydroxy($C_2$-$C_7$)alkyle ; un formyle ; un ($C_1$-$C_7$)alkylcarbonyle ; un benzoyle ; un ω-carboxy($C_1$-$C_7$)alkyle ; un ω-($C_1$-$C_7$)alcoxycarbonyl($C_1$-

$C_7$)alkyle ; un ω-benzyloxycarbonyl($C_1$-$C_7$)alkyle ; un ω-amino($C_2$-$C_7$)alkyle dans lequel le groupe amino est libre ou substitué par un ou deux ($C_1$-$C_7$)alkyles ou sous forme d'ion ammonium ; un ω-carbamoyl($C_1$-$C_7$)alkyle dans lequel le carbamoyle est libre ou substitué par un ou deux ($C_1$-$C_7$)alkyles ;

- $R_8$ et $R_9$ représentent chacun indépendamment un hydrogène ; un ($C_1$-$C_7$)alkyle ; un groupe -$CH_2$-Ar ; $R_9$ peut de plus représenter un groupe -Ar ; un ($C_3$-$C_8$)alcényle ; un formyle ; un ($C_1$-$C_7$)alkylcarbonyle ; un ($C_1$-$C_7$)alkylthiocarbonyle ; un ($C_3$-$C_7$)cycloalkylcarbonyle ; un ($C_3$-$C_7$)cycloalkylthiocarbonyle un ω-amino($C_2$-$C_7$)alkylcarbonyle dans lequel l'amino est libre ou substitué par un ou deux ($C_1$-$C_7$)alkyles ; un ω-hydroxy($C_1$-$C_7$)alkylcarbonyle ; un ω-benzyloxy($C_1$-$C_7$)alkylcarbonyle ; un pyridylcarbonyle ; un méthylpyridylcarbonyle ; un thiénylcarbonyle ; un groupe -CO-Ar ; un groupe-CO-$CH_2$-Ar ; un ($C_1$-$C_7$)alcoxycarbonyle ; un phénoxycarbonyle ; un phénoxythiocarbonyle ; un benzyloxycarbonyle ; un groupe -CO-$CR_{10}R_{23}$-$NR_{11}R_{19}$, un groupe -$CR_{10}R_{23}COR_{12}$, un groupe -$(CH_2)_tCOR_{12}$ ; un groupe -$CO(CH_2)_uCOR_{12}$ ; un groupe -$CONR_{14}R_{24}$ ; un groupe -$CSNR_{14}R_{24}$ ; un radical hétérocyclique choisi parmi pyrazolyle, imidazolyle, triazolyle, tétrazolyle, pyridyle, pyridazinyle, pyrimidinyle, thiazolyle ;
- ou bien $R_8$ et $R_9$ ensemble avec l'atome d'azote auquel ils sont liés constituent l'hydantoïne, la N-méthylhydantoïne, ou un radical hétérocyclique choisi parmi le pyrrol-1-yle, le Δ3-pyrrolin-1-yle, le pyrrolidin-1-yle, l'isoindolin-2-yle dans lequel le noyau benzénique est non substitué ou substitué par un halogène, un ($C_1$-$C_7$)alkyle, un trifluorométhyle ou un méthoxy ;
- $R_{10}$ et $R_{23}$ représentent chacun indépendamment l'hydrogène ; un ($C_1$-$C_7$)alkyle ; un benzyle ; ou $R_{10}$ et $R_{23}$ ensemble avec l'atome de carbone auquel ils sont liés constituent un ($C_3$-$C_7$)cycloalkyle ;
- $R_{11}$ et $R_{19}$ représentent chacun indépendamment l'hydrogène ou un ($C_1$-$C_7$)alkyle ;
- $R_{12}$ représente un hydroxy ; un ($C_1$-$C_7$)alcoxy ; un amino libre ou substitué par un ou deux ($C_1$-$C_7$)alkyles ;
- $R_{13}$ représente l'hydrogène ; un ($C_1$-$C_7$)alkyle ; un phényle ; un benzyle ; un formyle ; un ($C_1$-$C_7$)alkylcarbonyle ; un benzoyle ; un ($C_1$-$C_7$)alcoxycarbonyle ; un carbamoyle non substitué ou substitué par un ou deux ($C_1$-$C_7$)alkyles ;
- $R_{14}$ et $R_{24}$ représentent chacun indépendamment l'hydrogène ; un ($C_1$-$C_7$)alkyle ; $R_{24}$ peut de plus représenter un ($C_1$-$C_7$)alkyle substitué par $R_{15}$ ; un groupe -Ar ; un ($C_3$-$C_7$)cycloalkyle ; un adamantyle ;
- ou $R_{14}$ et $R_{24}$ ensemble avec l'atome d'azote auquel ils sont liés constituent un hétérocycle choisi parmi : la morpholine, la thiomorpholine, la pipérazine, l'azétidine, la pyrrolidine, la pipéridine ou la perhydroazépine, ledit hétérocycle étant non substitué ou substitué par un ou plusieurs groupes méthyles, par un phényle, par un groupe amino libre ou portant un groupe protecteur ;
- $R_{15}$ représente un groupe Ar ; un pyridyle ; un hydroxy ; un ($C_1$-$C_7$)alcoxy ; un groupe -$NR_{11}R_{19}$ ; un carboxy ; un ($C_1$-$C_7$)alcoxycarbonyle ;
- $R_{16}$ et $R_{22}$ représentent chacun indépendamment un hydrogène ou un ($C_1$-$C_7$)alkyle ; ou $R_{16}$ et $R_{22}$ ensemble avec l'atome d'azote auquel ils sont liés constituent un hétérocycle choisi parmi : l'azétidine, la pyrrolidine, la pipéridine, la pipérazine ou la perhydroazépine, le dit hétérocycle étant non substitué ou substitué par un ou plusieurs groupes méthyles ;
- $R_{17}$ et $R_{18}$ représentent chacun indépendamment l'hydrogène ou un ($C_1$-$C_8$)alkyle ; $R_{18}$ peut de plus représenter un ($C_3$-$C_7$)cycloalkyle non substitué ou substitué par un ($C_1$-$C_4$)alkyle ; un groupe -Ar ; un pyridyle ; un méthylpyridyle ; un pipérid-4-yle substitué en position 1 par $R_{27}$ ; un pipérid-1-yle ; un pyrrolidin-1-yle ; un morpholin-4-yle ; un ($C_1$-$C_7$)alkyle substitué par un ou plusieurs halogènes ou par $R_{26}$.
- ou bien $R_{17}$ et $R_{18}$ ensemble avec l'atome d'azote auquel ils sont liés constituent un radical hétérocyclique $R_{25}$ ;
- $R_{20}$ et $R_{21}$ représentent chacun indépendamment l'hydrogène ou un ($C_1$-$C_7$)alkyle ;
- ou bien $R_{20}$ et $R_{21}$ ensemble avec l'atome d'azote auquel ils sont liés constituent un radical hétérocyclique $R_{25}$ ;
- $R_{25}$ représente un morpholin-4-yle ; un thiomorpholine-4-yle ; un azétidin-1-yle non substitué ou substitué en position 3 par un groupe -$NR_{11}R_{19}$, un ($C_1$-$C_7$)alkyle, un phényle, un benzyle ou un ($C_1$-$C_7$)alkylcarbonyle ; un perhydroazépin-1-yle ; un pipérazin-1-yle non substitué ou substitué en position 4 par un ($C_1$-$C_7$)alkyle, un phényle, un benzyle, un ($C_1$-$C_7$)alkylcarbonyle, un ($C_1$-$C_7$)alcoxycarbonyle ou un benzyloxycarbonyle ; un pipérid-1-yle non substitué ou substitué en position 4 par un ($C_1$-$C_7$)alkyle, un phényle, un benzyle, un ($C_1$-$C_7$)alkylcarbonyle, un groupe -$NR_{11}R_{19}$ ; un pyrrolidin-1-yle non substitué ou substitué par un ($C_1$-$C_7$)alkyle, un phényle, un benzyle, un ($C_1$-$C_7$)alkylcarbonyle, un hydroxyméthyle, un carboxy, un ($C_1$-$C_7$)alcoxycarbonyle, un carbamoyle non substitué ou substitué par un ou deux ($C_1$-$C_7$)alkyles ;
- $R_{26}$ représente un hydroxy ; un ($C_1$-$C_7$)alcoxy ; un cyano ; un carboxy ; un ($C_1$-$C_7$)alcoxycarbonyle ; un benzyloxycarbonyle ; un groupe -$NR_{11}R_{19}$ ; un carbamoyle libre ou substitué par un ou deux ($C_1$-$C_7$)alkyles ; un pyrrolidin-1-ylcarbonyle ; un pipérid-1-ylcarbonyle ; un perhydroazépin-1-ylcarbonyle ; un grou-

pe -Ar ; un ($C_3$-$C_7$)cycloalkyle ; un adamantyle ; un radical hétérocyclique choisi parmi un pyridyle, un méthylpyridyle, un furanyle, un tétrahydrofuranyle, un thiényle, un méthylthiényle, un pyrrolidin-1-yle, un pipérid-1-yle, un perhydroazépin-1-yle ;

- $R_{27}$ représente un hydrogène ; un ($C_1$-$C_7$)alkyle ; un phényle ; un benzyle ; un formyle ; un ($C_1$-$C_7$)alkylcarbonyle ; un benzoyle ; un ($C_1$-$C_7$)alcoxycarbonyle ; un phénoxycarbonyle ; un carbamoyle libre ou substitué par un ou deux ($C_1$-$C_7$)alkyles.
- Ar représente un phényle non substitué ou substitué une ou plusieurs fois par un substituant choisi parmi : un atome d'halogène, un ($C_1$-$C_7$)alkyle, un trifluorométhyle, un hydroxy, un ($C_1$-$C_7$)alcoxy, un carboxy, un ($C_1$-$C_7$)alcoxycarbonyle, un ($C_1$-$C_7$)alkylcarbonyloxy, un nitro, un cyano, un amino, un ($C_1$-$C_7$)alkylamino, un ($C_1$-$C_7$)dialkylamino, lesdits substituants étant identiques ou différents ;
- m est 1 ou, lorsque $R_6$ est un halogène, un ($C_1$-$C_7$)alkyle ou un ($C_1$-$C_7$)alcoxy, m peut également être 2, 3 ou 4 ou bien $(R_6)_m$ peut représenter m substituants ayant différentes valeurs choisies parmi halogène, ($C_1$-$C_7$)alkyle ou ($C_1$-$C_7$)alcoxy ;
- p et q représentent chacun un nombre entier, leur somme peut varier de 3 à 6;
- t représente un nombre entier qui peut varier de 2 à 5 ;
- u représente un nombre entier qui peut varier de 0 à 3 ;
- X représente l'oxygène, un groupe $NR_{13}$, un groupe $N(O)R_{13}$, un groupe $S(O)_X$ ;
- x représente 0, 1 ou 2 ;

ainsi que leurs sels éventuels.

Avantageusement les composés de l'invention sont les composés de formule (I) dans laquelle :

- $R_1$ et $R_2$ représentent chacun indépendamment un hydrogène ; un halogéno ; un hydroxy ; un ω-halogéno($C_1$-$C_7$)alcoxy ; un ($C_1$-$C_7$)alkyle ; un trifluorométhyle ; un ($C_1$-$C_7$)alcoxy ; un polyhalogéno($C_1$-$C_7$)alcoxy ; un ω-hydroxy($C_2$-$C_7$)alcoxy, un ω-méthoxy($C_2$-$C_7$)alcoxy ; un ω-amino($C_2$-$C_7$)alcoxy libre ou substitué par un ou deux ($C_1$-$C_7$)alkyles ; un ($C_3$-$C_7$)cycloalkyloxy ; un ($C_3$-$C_7$)cycloalkylméthoxy ; un phénoxy ; un benzyloxy ; un ($C_1$-$C_7$)alkylthio ; un phénylthio ; un nitro ; un amino libre ou substitué par un ou deux ($C_1$-$C_7$)alkyles ; un cyano ; un formyle ; un ($C_1$-$C_6$)alkylcarbonyle ; un benzoyle ; un formyloxy ; un ($C_1$-$C_6$)alkylcarbonyloxy ; un benzoyloxy ; un ($C_1$-$C_7$)alkylsulfonamido ; un phénylsulfonamido ; un benzylsulfonamido ; un ($C_1$-$C_7$)alkylcarbonylamino ; un ($C_1$-$C_7$)alcoxycarbonylamino ; un uréido non substitué ou substitué par un phényle, un benzyle ou par un ou deux ($C_1$-$C_7$)alkyles ; un thiouréido non substitué ou substitué par un phényle, un benzyle ou par un ou deux ($C_1$-$C_7$)alkyles ; à la condition que $R_1$ et $R_2$ ne soient pas simultanément l'hydrogène ;
- $R_3$ et $R_4$ ensemble constituent un groupe $-(CH_2)_pX(CH_2)_q-$ ;

  ou
- $R_3$ et $R_4$ ensemble avec le carbone auquel ils sont liés constituent un cycle hydrocarboné en $C_3$-$C_{12}$, saturé ou insaturé, éventuellement condensé, non substitué ou substitué par un ou plusieurs groupes ($C_1$-$C_7$)alkyles, par un spirocycloalkyle en $C_3$-$C_5$, par un groupe oxo, par un hydroxy libre ou substitué par un groupe choisi parmi les groupes : ($C_1$-$C_4$)alkyle, ($C_1$-$C_2$)alcoxy($C_1$-$C_4$)alkyle, phényl($C_1$-$C_2$)alcoxy($C_1$-$C_4$)alkyle, tétrahydrofuranyle et tétrahydropyranyle ;
- $R_5$ représente l'hydrogène ou l'une des valeurs désignées pour $R_5$ ;
- $R_5$ représente un halogène ; un ($C_1$-$C_7$)alkyle ; un trifluorométhyle ; un cyano ; un nitro ; un hydroxylamino ; un hydroxy ; un carboxy ; un guanidino non substitué ou substitué en position 1 par un ($C_1$-$C_7$)alkyle et/ou en position 3 par un ou deux ($C_1$-$C_7$)alkyles, un phényle ou un benzyle ; un groupe $OR_7$ ; un groupe $SR_7$ ; un formyle ; un ($C_1$-$C_6$)alkylcarbonyle ; un benzoyle ; un ($C_1$-$C_7$)alcoxycarbonyle ; un phénoxycarbonyle ; un benzyloxycarbonyle ; un groupe $-CONR_{17}R_{18}$ ; un groupe $-CSNR_{11}R_{19}$ ; un groupe $-SO_2NR_{20}R_{21}$ ; un ($C_1$-$C_7$)alkylsulfonamido ; un groupe $-NHSO_2$-Ar ; un benzylsulfonamido ; un aminosulfonamido dans lequel l'amino est libre ou substitué par $R_{16}$ et $R_{22}$ ; un groupe $-NR_8R_9$ ; un groupe $-CO-NH-CR_{10}R_{23}-COR_{12}$ ; un groupe $-CH_2NH_2$ libre ou substitué par un ou deux ($C_1$-$C_7$) alkyles ;
- $R_7$ représente un ($C_1$-$C_7$)alkyle ; un phényle ; un benzyle ; un ($C_3$-$C_7$)cycloalkyle ; un ($C_2$-$C_7$)alcényle ; un ω-halogéno($C_2$-$C_7$)alkyle ; un polyhalogéno($C_1$-$C_7$)alkyle ; un ω-hydroxy($C_2$-$C_7$)alkyle ; un formyle ; un ($C_1$-$C_6$)alkylcarbonyle ; un benzoyle ; un ω-carboxy($C_1$-$C_7$)alkyle ; un ω-($C_1$-$C_7$)alcoxycarbonyl($C_1$-$C_7$)alkyle ; un ω-benzyloxycarbonyl($C_1$-$C_7$)alkyle ; un ω-amino($C_2$-$C_7$)alkyle dans lequel le groupe amino est libre ou substitué par un ou deux ($C_1$-$C_7$)alkyles ou sous forme d'ion ammonium ; un ω-carbamoyl($C_1$-$C_7$)alkyle dans lequel le carbamoyle est libre ou substitué par un ou deux ($C_1$-$C_7$)alkyles ;
- $R_5$ et $R_9$ représentent chacun indépendamment un hydrogène ; un ($C_1$-$C_7$)alkyle ; un groupe $-CH_2$-Ar ; $R_9$ peut de plus représenter un groupe -Ar ; un ($C_3$-$C_8$)alcényle ; un formyle ; un ($C_1$-$C_6$)alkylcarbonyle ; un ($C_1$-$C_6$)alkylthiocarbonyle ; un ($C_3$-$C_7$)cycloalkylcarbonyle ; un ($C_3$-$C_7$)cycloalkylthiocarbonyle ; un ω-amino($C_2$-$C_6$)alkylcarbonyle ; un ω-hydroxy($C_1$-$C_6$)alkylcarbonyle ; un ω-benzyloxy($C_1$-$C_6$)alkylcarbonyle ; un pyridylcarbonyle ; un méthylpyridylcarbonyle ; un thiénylcarbonyle ; un groupe -CO-Ar ; un grou-

pe-CO-$CH_2$-Ar ; un ($C_1$-$C_7$)alcoxycarbonyle ; un phénoxycarbonyle ; un groupe -CO-$CR_{10}R_{23}$-$NR_{11}R_{19}$, un groupe -$CR_{10}R_{23}COR_{12}$, un groupe -$(CH_2)_tCOR_{12}$ ; un groupe -$CO(CH_2)_uCOR_{12}$ ; un groupe -$CONR_{14}R_{24}$ ; un groupe -$CSNR_{14}R_{24}$ ; un radical hétérocyclique choisi parmi pyrazolyle, imidazolyle, triazolyle, tétrazolyle, pyridyle, pyridazinyle, pyrimidinyle, thiazolyle ;

- ou bien $R_8$ et $R_9$ ensemble avec l'atome d'azote auquel ils sont liés constituent l'hydantoïne, ou un radical hétérocyclique choisi parmi le pyrrol-1-yle, le Δ3-pyrrolin-1-yle, le pyrrolidin-1-yle, l'isoindolin-2-yle dans lequel le noyau benzénique est non substitué ou substitué par un halogène, un ($C_1$-$C_7$)alkyle, un trifluorométhyle ou un méthoxy ;
- $R_{10}$ et $R_{23}$ représentent chacun indépendamment l'hydrogène ; un ($C_1$-$C_7$)alkyle ; un benzyle ; ou $R_{10}$ et $R_{23}$ ensemble avec l'atome de carbone auquel ils sont liés constituent un ($C_3$-$C_7$)cycloalkyle ;
- $R_{11}$ et $R_{19}$ représentent chacun indépendamment l'hydrogène ou un ($C_1$-$C_7$)alkyle ;
- $R_{12}$ représente un hydroxy ; un ($C_1$-$C_7$)alcoxy ; un amino libre ou substitué par un ou deux ($C_1$-$C_7$)alkyles ;
- $R_{13}$ représente l'hydrogène ; un ($C_1$-$C_7$)alkyle ; un phényle ; un benzyle ; un formyle ; un ($C_1$-$C_6$)alkylcarbonyle ; un benzoyle ; un ($C_1$-$C_7$)alcoxycarbonyle ; un carbamoyle non substitué ou substitué par un ou deux ($C_1$-$C_7$)alkyles ;
- $R_{14}$ et $R_{24}$ représentent chacun indépendamment l'hydrogène ; un ($C_1$-$C_7$)alkyle ; $R_{24}$ peut de plus représenter un ($C_1$-$C_7$)alkyle substitué par $R_{15}$ ; un phényle non substitué ou substitué par un hydroxy, un amino, un ($C_1$-$C_7$)alkylamino, un ($C_1$-$C_7$)dialkylamino ; un ($C_3$-$C_7$)cycloalkyle ; un adamantyle ;
- ou $R_{14}$ et $R_{24}$ ensemble avec l'atome d'azote auquel ils sont liés constituent un hétérocycle choisi parmi : la morpholine, la thiomorpholine, la pipérazine, l'azétidine, la pyrrolidine ou la pipéridine, ledit hétérocycle étant non substitué ou substitué par un ou plusieurs groupes méthyles, par un phényle, par un groupe amino libre ou portant un groupe protecteur ;
- $R_{15}$ représente un groupe -Ar ; un pyridyle ; un hydroxy ; un ($C_1$-$C_7$)alcoxy ; un groupe -$NR_{11}R_{19}$ ; un carboxy ; un ($C_1$-$C_6$)alcoxycarbonyle ;
- $R_{16}$ et $R_{22}$ représentent chacun indépendamment un hydrogène ou un ($C_1$-$C_7$)alkyle ; ou $R_{16}$ et $R_{22}$ ensemble avec l'atome d'azote auquel ils sont liés constituent un hétérocycle choisi parmi : l'azétidine, la pyrrolidine, la pipéridine, la pipérazine ; ledit hétérocycle étant non substitué ou substitué par un ou plusieurs groupes méthyles ;
- $R_{17}$ et $R_{18}$ représentent chacun indépendamment l'hydrogène ou un ($C_1$-$C_7$)alkyle ; $R_{18}$ peut de plus représenter un groupe -Ar ; un pyridyle ; un méthylpyridyle ; un pipérid-4-yle ; un méthylpipérid-4-yle ; un ($C_1$-$C_7$)alkyle substitué par un ou plusieurs halogènes ou par $R_{26}$.
- ou bien $R_{17}$ et $R_{18}$ ensemble avec l'atome d'azote auquel ils sont liés constituent un radical hétérocyclique $R_{25}$ ;
- $R_{20}$ et $R_{21}$ représentent chacun indépendamment l'hydrogène ou un ($C_1$-$C_7$)alkyle ;
- ou bien $R_{20}$ et $R_{21}$ ensemble avec l'atome d'azote auquel ils sont liés constituent un radical hétérocyclique $R_{25}$ ;
- $R_{25}$ représente un azétidin-1-yle non substitué ou substitué en position 3 par un groupe -$NR_{11}R_{19}$, un ($C_1$-$C_7$)alkyle, un phényle, un benzyle ou un ($C_1$-$C_6$)alkylcarbonyle ; un pipérazin-1-yle non substitué ou substitué en position 4 par un ($C_1$-$C_7$)alkyle, un phényle, un benzyle, un ($C_1$-$C_6$)alkylcarbonyle, un pipérid-1-yle non substitué ou substitué en position 4 par un ($C_1$-$C_7$)alkyle, un phényle, un benzyle, un ($C_1$-$C_6$)alkylcarbonyle, un groupe -$NR_{11}R_{19}$ ; un pyrrolidin-1-yle non substitué ou substitué par un ($C_1$-$C_7$)alkyle, un phényle, un benzyle, un ($C_1$-$C_7$)alkylcarbonyle, un hydroxyméthyle, un carboxy, un ($C_1$-$C_7$)alcoxycarbonyle, un carbamoyle non substitué ou substitué par un ou deux ($C_1$-$C_7$)alkyles ;
- $R_{26}$ représente un hydroxy ; un ($C_1$-$C_7$)alcoxy ; un cyano ; un carboxy ; un ($C_1$-$C_7$)alcoxycarbonyle ; un benzyloxycarbonyle ; un groupe -$NR_{11}R_{19}$ ; un carbamoyle libre ou substitué par un ou deux ($C_1$-$C_7$)alkyles ; un pyrrolidin-1-ylcarbonyle ; un pipérid-1-ylcarbonyle ; un groupe -Ar ; un ($C_3$-$C_7$)cycloalkyle ; un adamantyle ; un radical hétérocyclique choisi parmi un pyridyle, un méthylpyridyle, un furanyle, un tétrahydrofuranyle, un thiényle, un méthylthiényle, un pyrrolidin-1-yle, un pipérid-1-yle ;
- Ar représente un phényle non substitué ou substitué une ou plusieurs fois par un substituant choisi parmi : un atome d'halogène, un ($C_1$-$C_7$)alkyle, un trifluorométhyle, un ($C_1$-$C_7$)alcoxy, un carboxy, un ($C_1$-$C_7$)alcoxycarbonyle, un ($C_1$-$C_7$)alkylcarbonyloxy ;
- m est 1 ou, lorsque $R_6$ est un halogène, un ($C_1$-$C_7$)alkyle ou un ($C_1$-$C_7$)alcoxy, m peut également être 2, 3 ou 4 ou bien $(R_6)_m$ peut représenter m substituants ayant différentes valeurs choisies parmi halogène, ($C_1$-$C_7$)alkyle ou ($C_1$-$C_7$)alcoxy ;
- p et q représentent chacun un nombre entier, leur somme peut varier de 3 à 6 ;
- t représente un nombre entier qui peut varier de 2 à 5 ;
- u représente 2 ou 3 ;

- X représente l'oxygène, un groupe $NR_{13}$, un groupe $N(O)R_{13}$, un groupe $S(O)_X$ ;
- x représente 0, 1 ou 2 ;

ainsi que leurs sels éventuels.

Lorsqu'un composé selon l'invention présente un ou plusieurs carbones asymétriques, l'invention comprend tous les isomères optiques de ce composé.

Les sels des composés de formule (I) selon la présente invention comprennent ceux avec des acides minéraux ou organiques qui permettent une séparation ou une cristallisation convenable des composés de formule (I), tels que l'acide picrique, l'acide oxalique ou un acide optiquement actif, par exemple un acide mandélique ou un acide camphosulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalènesulfonate.

Les sels des composés de formule (I) comprennent également les sels avec des bases organiques ou minérales, par exemple les sels des métaux alcalins ou alcalino-terreux comme les sels de sodium, de potassium, de calcium, les sels de sodium et de potassium étant préférés, ou avec une amine, telle que le trométamol, ou bien les sels d'arginine, de lysine, ou de toute amine physiologiquement acceptable.

Selon la présente invention, par halogène on entend un atome choisi parmi le fluor, le chlore, le brome ou l'iode, de préférence le fluor ou le chlore.

Selon la présente invention par groupe protecteur du groupe amino on entend un groupe tel que : un $(C_1-C_4)$alkyle, par exemple un méthyle ou un *tert*-butyle ; un benzyle ; un benzyle substitué tel que p-nitrobenzyle, p-chlorobenzyle, p-méthoxybenzyle ; un benzhydryle ; un trityle ; un benzoyle ; un $(C_1-C_4)$alkylcarbonyle, par exemple un acétyle ; un $(C_1-C_4)$alcoxycarbonyle, par exemple un méthoxycarbonyle, un éthoxycarbonyle ou un *tert*-butoxycarbonyle ; un benzyloxycarbonyle.

Selon la présente invention par alkyle en $C_1-C_7$ ou respectivement en $C_1-C_8$ ou en $C_1-C_4$, on entend un alkyle droit ou ramifié en $C_1-C_7$ ou respectivement en $C_1-C_8$ ou en $C_1-C_4$. Par alcoxy en $C_1-C_7$ ou respectivement en $C_1-C_4$ on entend un alcoxy droit ou ramifié en $C_1-C_7$ ou respectivement en $C_1-C_4$.

Selon la présente invention par cycle hydrocarboné en $C_3-C_{12}$ éventuellement condensé, saturé ou insaturé, on entend différents cycles hydrocarbonés de structure monocyclique, bicyclique ou tricyclique, par exemple un cyclobutane, un cyclopentane, un cyclohexane, un cycloheptane, un cyclooctane, un indane, un hexahydroindane, un adamantane, un norbornane, un norbornène, un dihydrophénalène, un tricyclo $[5.2.1.0^{2,6}]$ décane ou un tricyclo $[5.2.1.0^{2,6}]$dec-8-ène, un bicyclo [2.2.1]heptane, un bicyclo [3.3.1]nonane.

Les composés de formule (I) dans lesquels $R_1$ est en position 5 du 1,3-dihydroindol-2-one et $R_2$ est l'hydrogène sont des composés préférés.

Les composés de formule (I) dans lesquels $R_1$ est un atome de chlore ou de fluor ou un groupe éthoxy en position 5 du 1,3-dihydro-indol-2-one et $R_2$ est l'hydrogène sont des composés préférés.

Les composés de formule (I) dans lesquels $R_3$ et $R_4$ ensemble avec le carbone auquel ils sont liés constituent un cycle hydrocarboné en $C_3-C_{12}$ sont des composés préférés ; particulièrement préférés sont les composés dans lesquels $R_3$ et $R_4$ ensemble avec le carbone auquel ils sont liés constituent un cycloheptane, un adamantane, un tricyclo $[5.2.1.0^{2,6}]$dec-8-ène, un tricyclo $[5.2.1.0^{2,6}]$décane, un bicyclo [2.2.1]heptane, un bicyclo [3.3.1]nonane ou un cyclohexane non substitué ou substitué par un spirocycloalkyle en $C_3-C_5$, par un ou deux groupes alkyles en $C_1-C_7$, ou par un alcoxy en $C_1-C_4$.

On préfère tout particulièrement les composés de formule (I) dans lesquels $R_3$ et $R_4$ ensemble avec le carbone auquel ils sont liés constituent un tricyclo $[5.2.1.0^{2,6}]$décane ou un tricyclo$[5.2.1.0^{2,6}]$dec-8-ène.

Les composés de formule (I) dans lesquels $R_3$ et $R_4$ ensemble avec le carbone auquel ils sont liés constituent un cycle pipéridine-4 non substitué ou substitué sur l'azote par un alkyle en $C_1-C_7$ sont également préférés.

Les composés de formule (I) dans laquelle $R_5$ est soit l'hydrogène soit un groupe méthoxy en position 2 et $R_5$ en position 4 est un groupe $-NR_5R_9$, $R_8$ et $R_9$ étant tels que définis précédemment sont également des composés préférés de l'invention.

Particulièrement préférés sont les composés de formule (I) dans lesquels $R_5$ est soit l'hydrogène soit un groupe de méthoxy en position 2 et $R_6$ en position 4 représente un méthoxy, un $(C_1-C_7)$alkylcarboxamido, un groupe -NHCO-Ar, un groupe $-CONR_{17}R_{18}$, ou un groupe $-NR_8CONR_{14}R_{24}$ dans lesquels les substituants Ar, $R_{17}$, $R_{18}$, $R_8$, $R_{14}$ et $R_{24}$ sont tels que définis ci-dessus pour les composés de formule (I).

Dans la description et dans les exemples, les abréviations suivantes sont utilisées.

DCM : dichlorométhane
Ether : éther éthylique
Ether iso : éther isopropylique
Boc : *tert*-butoxycarbonyle
Me, MeO : méthyle, méthoxy

Et, OEt : éthyle, éthoxy
Pr, iPr, nPr : propyle, isopropyle, n-propyle
Bu, iBu, tBu : butyle, isobutyle, *tert*-butyle
Ts : tosyle
Ph : phényle
Bz : benzyle
Ac : acétyle
AcOEt : acétate d'éthyle
AcOH : acide acétique
MeOH : méthanol
EtOH : éthanol
DMF : diméthylformamide
THF : tétrahydrofuranne
DMSO : diméthylsulfoxyde
DIPEA : diisopropyléthylamine
TEA : triéthylamine
TFA : acide trifluoroacétique
TMEDA : tétraméthyléthylènediamine
BOP : hexafluorophosphate de benzotriazol-1-yloxy-tris(dimethylaminophosphonium)
Réactif de Lawesson : 2,4-bis(4-méthoxyphényl)-1,3-dithia-2,4- diphosphétane-2,4-disulfure
F : point de fusion
Solution saline : eau saturée en chlorure de sodium
CCM : chromatographie en couche mince
HPLC : chromatographie liquide à haute pression
Eau chlorhydrique : acide chlorhydrique dilué, environ 1N
TA : température ambiante

La présente invention a également pour objet un procédé de préparation des composés selon l'invention caractérisé en ce que :

a) on fait agir sur un 1,3-dihydro-indol-2-one disubstitué en position 3, de formule :

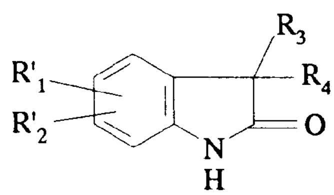

(II)

dans laquelle R'$_1$ et R'$_2$ représentent, respectivement, soit $R_1$ et $R_2$ tels que définis pour (I), soit des groupes précurseurs de $R_1$, $R_2$, et $R_3$, $R_4$ sont tels que définis ci-dessus pour (I), un halogénure de benzyle de formule :

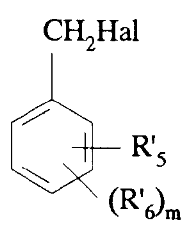

(III)

dans laquelle Hal représente un atome d'halogène, de préférence le brome ou le chlore et R'$_5$ et R'$_6$ représentent, respectivement, soit $R_5$ et $R_6$ tels que définis ci-dessus pour (I), soit des groupes précurseurs de $R_5$ et $R_5$ ; et,

b) soit, lorsque R'$_1$ = $R_1$, R'$_2$ = $R_2$, R'$_5$ = $R_5$ et R'$_6$ = $R_6$, on isole le composé de formule (I) ainsi obtenu ;

c) soit, lorsque l'un quelconque des groupes R'$_1$, R'$_2$, R'$_5$ et/ou R'$_6$ représente respectivement un groupe précurseur de $R_1$, $R_2$, $R_5$ et/ou $R_6$, on soumet le composé obtenu à l'étape a), ci-après dénommé composé (I'), à un traitement ultérieur pour préparer le composé de formule (I) par transformation de l'un quelconque

des groupes $R'_1$, $R'_2$, $R'_5$ et/ou $R'_6$ en, respectivement, $R_1$, $R_2$, $R_5$ et/ou $R_6$.

La réaction de l'étape a), est effectuée dans un solvant anhydre tel que le DMF ou le THF, en présence d'un hydrure métallique tel que l'hydrure de sodium par exemple, ou en présence d'un alcoolate comme le *tert*-butylate de potassium.

Les 1,3-dihydro-indol-2-one (II) sont connus ou peuvent être préparés selon différents modes opératoires par des méthodes connues.

Des composés (II) dans lesquels $R'_1$ et/ou $R'_2$ représentent un halogène et $R_3$, $R_4$ ensemble avec le carbone auquel ils sont liés constituent un spirocyclobutane, un spirocyclohexane ou un spirocycloheptane sont connus, par exemple dans D.W.Robertson et al., J.Med.Chem., 1987, 30 (5),824-829. De plus, le 5-chloro-3-spirocyclopentaneindol-2-one est décrit dans le brevet US 3 947 451.

Pour la préparation des composés (II), lorsque $R_3$ et $R_4$ représentent ensemble un groupement hydrocarboné, on peut utiliser la réaction de Brunner décrite par R.F. Moore et S.G.P. Plant dans J. Chem. Soc., 1951, 3475-3478, et qui conduit à la préparation de composés (II) dans lesquels $CR_3R_4$ représente un cyclopentane ou un cyclohexane.

Cette réaction est effectuée par cyclisation d'un dérivé de phénylhydrazide de formule :

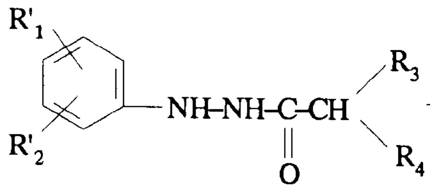

(IV)

dans laquelle $R'_1$ et $R'_2$ sont tels que définis ci-dessus pour (II) et $R_3$, $R_4$ ont les définitions données ci-dessus pour (I), par exemple par chauffage dans la quinoléine en présence d'oxyde de calcium.

Le dérivé de phénylhydrazide (IV) est obtenu selon les mêmes auteurs par action d'un dérivé de l'hydrazine de formule :

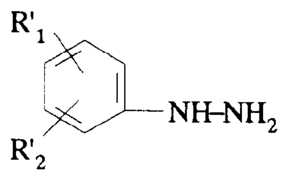

(V)

dans laquelle $R'_1$ et $R'_2$ ont les définitions données ci-dessus pour (II), sur un halogénure d'acide de formule :

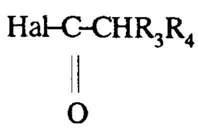

(VI)

dans laquelle $R_3$ et $R_4$ ont les définitions données ci-dessus pour (I).

Selon un mode de réalisation particulier, lorsque $R_3$ et $R_4$ ensemble avec le carbone auquel ils sont liés constituent un cycle hydrocarboné polycondensé, par exemple un norbornane ou un norbornène, on opère d'après la méthode décrite par J. Wolff et al., Tetrahedron, 1986, 42 (15), 4267-4272 : on prépare d'abord un sel de lithium du composé (IV) par action d'un réactif lithié tel que le n-butyllithium, dans un solvant inerte tel que le THF, à basse température, puis on effectue la cyclisation par chauffage dans un solvant tel que le naphtalène ou le prehnitène (1,2,3,4-tétraméthylbenzène).

Les composés (II) dans lesquels $R'_1 = R'_2 = H$ et $CR_3R_4$ représente l'adamantane sont décrits par I. Fleming et al., J.Chem.Soc., Perkin Trans I, 1991, 3, 617-626. Les composés (II) dans lesquels $R_3$ et $R_4$ ensemble avec l'atome de carbone avec lequel ils sont liés constituent un adamantane et $R'_1$ et $R'_2$ sont différents de l'hydrogène peuvent être préparés par la méthode décrite ci-dessus.

Les dérivés de l'hydrazine (V) sont connus ou préparés par des méthodes connues. Il en est de même pour les halogènures d'acide (VI).

On peut également préparer un 1,3-dihydro-indol-2-one disubstitué en position 3 (II), à partir d'un 1,3-dihydro-indol-2-one de formule :

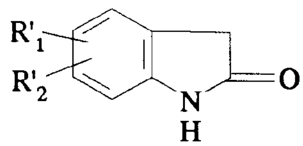

(VII)

dans laquelle R'$_1$ et R'$_2$ ont les valeurs définies ci-dessus pour (II) en utilisant différents procédés.

Par exemple, selon la méthode décrite par A.S. Kende et J.C. Hodges dans Synth. Commun., 1982, 12 (1), 1-10, on réalise l'addition d'un agent alkylant dans un solvant approprié. Ainsi pour préparer un composé (II) dans lequel $R_3$ et $R_4$ ensemble forment un groupe de formule $-(CH_2)_n-$ avec n variant de 3 à 12, on fait agir un composé de formule $Z(CH_2)_nZ$ dans laquelle Z représente un groupe nucléofuge tel qu'un halogène, de préférence le brome ou l'iode, un groupe tosyloxy ou un groupe mésyloxy.

On prépare de la même façon les composés (II) dans lesquels $R_3$ et $R_4$ ensemble avec le carbone auquel ils sont liés constituent un cycle hydrocarboné en $C_4$-$C_8$ substitué par un ou plusieurs groupes alkyles en $C_1$-$C_7$ ou par un spirocycloalkyle en $C_3$-$C_5$.

Lorsque $R_3$ et $R_4$ ensemble constituent un groupe $(CH_2)_p X(CH_2)_q-$, dans lequel p, q et X ont les valeurs données ci-dessus pour (I), on peut préparer un 1,3-dihydro-indol-2-one disubstitué en position 3 de formule (II), à partir d'un 1,3-dihydro-indol-2-one non substitué en 3 (VII) par action d'un composé de formule :

$$Z-(CH_2)_p-X-(CH_2)_q-Z \qquad (VIII)$$

dans laquelle Z est tel que défini ci-dessus et X, p et q ont les valeurs définies ci-dessus pour (I). La réaction est effectuée en présence d'un alcoolate, par exemple le *tert*-butylate de potassium, dans un solvant anhydre, tel que le THF par exemple.

Lorsque X représente un atome d'azote substitué par un formyle, un $(C_1$-$C_7)$alkylcarbonyle, un benzoyle, un $(C_1$-$C_7)$alcoxycarbonyle ou par un N-$(C_1$-$C_7)$alkylcarbamoyle, la substitution sur X peut être réalisée soit sur le dérivé 1,3-dihydro-indol-2-one (II), soit sur le composé final (I) à partir d'un composé dans lequel l'atome d'azote (X = NH) n'est pas substitué.

Ainsi lorsque X représente un atome d'azote substitué par un $(C_1$-$C_7$alcoxycarbonyle, on prépare d'abord un composé (II) ou (I) dans lequel X est NH, puis on fait agir le chloroformiate approprié pour obtenir le composé (II) ou (I) souhaité. De la même façon, on fait agir sur un composé (II) ou (I) dans lequel X = NH un isocyanate d'alkyle en $C_1$-$C_7$, pour obtenir un dérivé (II) ou un composé (I) dans lequel X représente un atome d'azote substitué par un N-$(C_1$-$C_7)$alkylcarbamoyle. On fait agir sur un composé (II) ou un composé (I) dans lequel X = NH l'acide formique en présence d'anhydride acétique ou respectivement un chlorure d'acide ou un anhydride pour préparer un composé de formule (II) ou de formule (I) dans lequel X représente un atome d'azote substitué par un formyle ou respectivement un $(C_1$-$C_7)$alkylcarbonyle ou par un benzoyle.

Lorsque X représente un atome de soufre ou un atome d'azote substitué par $R_{13}$, on peut également préparer d'abord un composé de formule :

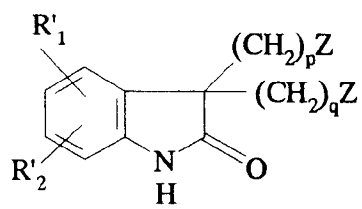

(II')

dans laquelle R'$_1$, R'$_2$, Z, p et q ont les définitions données ci-dessus, et effectuer une substitution nucléophile avec un sel de l'acide sulfhydrique ou une amine de formule $H_2NR_{13}$ dans un solvant tel qu'un alcool, un éther, le DMF, ou leur mélange, à une température comprise entre 5°C et la température de reflux.

Les 1,3-dihydro-indol-2-ones de formule (II') sont obtenus à partir des diols correspondants, protégés, par exemple par un groupe tétrahydropyran-2-yle. La réaction peut être effectuée selon J. Chem. Soc., Chem. Commun., 1989, 1619, par action du dibromotriphénylphosphorane.

Les composés (II) dans lesquels $R_3$ et $R_4$ ensemble avec le carbone auquel ils sont liés constituent un cycle pyrrolidine ou N-alkyl pyrrolidine, pipéridine ou N-alkyl pipéridine sont décrits par M.J. Komet dans J. Med. Chem., 1976, 19(7), 892-899.

En particulier, la horsfiline de formule :

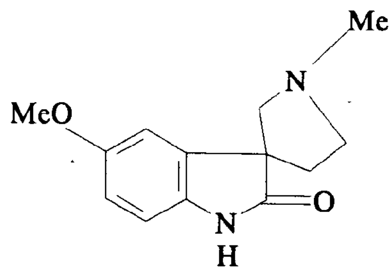

est un alcaloïde décrit dans A. Jossang et al., J. Org. Chem., 1991, 56 (23), 6527-6530.

Pour préparer un composé de formule (II) dans lequel $R_3$ et $R_4$ ensemble avec le carbone auquel ils sont liés constituent respectivement un tricyclo [5.2.1.0$^{2,6}$]décane ou un tricyclo [5.2.1.0$^{2,6}$]dec-8-ène, on fait agir sur un composé de formule (VII), respectivement un composé (VII)' ou un composé (VII)" de formules :

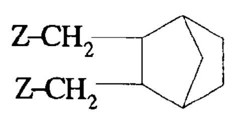

(VII)'

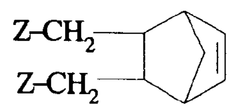

(VII)"

dans lesquelles Z est tel que défini ci-dessus. On utilise des composés (VII)' et (VII)" substitués par un ou plusieurs groupes alkyles en $C_1$-$C_7$ pour préparer des composés (II) dans lesquels lesdits carbocycles sont substitués.

On peut également préparer un composé de formule (II) dans lequel $R_3$ et $R_4$ ensemble avec le carbone auquel ils sont liés constituent un tricyclo[5.2.1.0$^{2,6}$] décane par hydrogénation catalytique par exemple en présence de palladium sur charbon ou de nickel de Raney® d'un composé de formule (II) dans lequel $R_3$ et $R_4$ ensemble avec le carbone auquel ils sont liés constituent un tricyclo[5-2-1-0$^{2,6}$]déc-8-ène.

On peut également préparer un composé de formule (I) dans lequel $R_3$ et $R_4$ ensemble avec le carbone auquel ils sont liés constituent un tricyclo [5.2.1.0$^{2,6}$]décane par hydrogénation catalytique par exemple en présence de palladium sur charbon ou de nickel de Raney ® d'un composé de formule (I) dans lequel $R_3$ et $R_4$ ensemble avec le carbone auquel ils sont liés constituent un tricyclo [5.2.1.0$^{2,6}$]dèc-8-ène.

Pour préparer un composé (II) dans lequel $R_3$ et $R_4$ ensemble avec le carbone auquel ils sont liés constituent respectivement un indane ou un hexahydroindane, on fait agir sur un composé (VII), respectivement un composé (VIII)' ou un composé (VIII)" de formules :

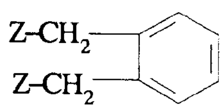

(VIII)'

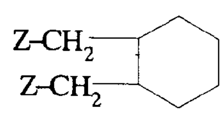

(VIII)"

dans lesquelles Z a la valeur indiquée ci-dessus pour (VIII). On utilise des composés (VIII)' et (VIII)" substitués par un ou plusieurs groupes alkyles en $C_1$-$C_7$ pour préparer des composés (II) dans lesquels l'indane ou l'hexahydroindane sont substitués.

De la même façon, on utilise la méthode de A.S. Kende et J.C. Hodges décrite ci-dessus pour préparer des composés de formule (II) dans lesquels les substituants $R_3$ et $R_4$ ensemble avec le carbone auquel ils sont liés constituent un cycle hydrocarboné en $C_3$-$C_{12}$, saturé ou insaturé, substitué par un ou plusieurs groupes ($C_1$-$C_7$)alkyles ou par un groupe choisi parmi : un groupe oxo, un spirocycloalkyle en $C_3$-$C_5$, un hydroxy substitué par un ($C_1$-$C_7$)alkyle, un $C_1$-$C_2$ alcoxy($C_1$-$C_4$)alkyle, un phényl($C_1$-$C_2$)alcoxy($C_1$-$C_4$)alkyle, un tétrahydrofuranyle, un tétrahydropyranyle. Pour obtenir les composés de formule (I) dans lesquels $R_3$ et $R_4$ ensemble avec le carbone auquel ils sont liés constituent un cycle hydrocarboné en $C_3$-$C_{12}$, saturé ou insaturé, substitué par un hydroxy, on effectue une déprotection des composés de formule (I) correspondants dans lesquels le groupe hydroxy est substitué par un ($C_1$-$C_2$) alcoxy($C_1$-$C_4$)alkyle un tétrahydrofuranyle ou un tétrahydropyranyle. Cette déprotection est effectuée en milieu acide, par exemple en présence d'un acide minéral ou organique, dans un solvant alcoolique ou éthérifié tel que le THF, à une température comprise entre 15°C et la

température de reflux ; par exemple la déprotection peut se faire en présence d'acide chlorhydrique ou de pyridinium toluènesulfonate dans un alcool.

Les composés de formule (II) dans lesquels $R_3$ et $R_4$ ensemble avec le carbone auquel ils sont liés constituent soit un cycle hydrocarboné en $C_3$-$C_{12}$, saturé ou insaturé, éventuellement condensé substitué par un groupe oxo, soit un groupe $-(CH_2)_p$-X-$(CH_2)_q$- avec X représentant un groupe SO, $SO_2$, ou $N(O)R_{13}$ sont préparés par des réactions d'oxydation connues à partir des composés correspondants de formule (II) dans lesquels $R_3$ et $R_4$ ensemble avec l'atome de carbone auquel ils sont liés constituent respectivement soit un cycle hydrocarboné en $C_3$-$C_{12}$, saturé ou insaturé, éventuellement condensé, substitué par un hydroxy, soit un groupe $-(CH_2)_p$-X-$(CH_2)_q$- avec X représentant un atome de soufre ou un groupe $NR_{13}$.

Par exemple, l'oxydation d'alcools secondaires en cétone peut s'effectuer en présence de complexes de l'oxyde de chrome tel que le pyridinium chlorochromate dans un solvant inerte tel que le chlorure de méthylène ou par des oxydants tels que le DMSO selon les méthodes décrites dans Tetrahedron, 1978, 34, 1651-1660.

L'oxydation des composés (II) comportant un atome de soufre ou d'azote (X = S, $NR_{13}$) peut s'effectuer en présence d'eau oxygénée ou de peracides tels que l'acide peracétique ou métachloroperbenzoïque dans des solvants inertes tels que les cétones ou l'acide acétique à des températures comprise entre 0°C et 50°C.

Les dérivés (VII) sont connus ou préparés par des méthodes connues. On peut citer par exemple J.V.RajanBabu dans J. Org. Chem., 1986, 51, 1704-1712.

Les composés de formule (II) portant certains substituants $R'_1$, $R'_2$ sur leur partie benzénique sont utilisés comme précurseurs pour la préparation de composés de formule (II) portant d'autres substituants $R'_1$, $R'_2$. Par exemple, les composés (II) dans lesquels $R'_1$ et/ou $R'_2$ = H peuvent être nitrés par les réactifs classiques ; ils peuvent également être acylés par action d'un chlorure d'acide de formule RCOCl dans lequel R représente un alkyle en $C_1$-$C_7$, en présence d'un acide de Lewis tel que le chlorure d'aluminium pour préparer un composé (II) dans lequel $R'_1$ et/ou $R'_2$ = COR. A partir d'un composé (II) dans lequel $R'_1$ est un groupe nitro et $R'_2$ est l'hydrogène, on prépare par hydrogénation catalytique le composé (II) dans lequel $R'_1$ est un groupe amino.

Les halogénures de formule (III) sont connus ou préparés par des méthodes connues.

A titre d'exemple, on peut citer des publications décrivant les dérivés d'halogénométhylbenzène suivants :

1,2,4- ou 4,2,1- ou 2,4,1- chlorométhylméthylméthoxybenzène : Bull. Soc. Chim., France, 1937.4, 1092.

2-chlorométhyl-1,3-diméthoxybenzène : Chem. Listy, 1953, 47, 601-612.

1-bromométhyl-2-méthoxy-4-nitrobenzène : Sci. Sinica (Peking), 1962, 11, 483-498.

1-bromométhyl-2-méthyl-4-nitrobenzène : Pharmazie, 1969, 24 (1), 29-32.

1-bromométhyl-2-méthoxy-4-nitrobenzène : Bull. Soc. Chim., France, 1962, 2255.

1-bromométhyl-4-méthoxy-2-nitrobenzène : Zh. Obshch. Khim., 1963, 33 (8), 2792-2793.

4-bromométhyl-3-méthoxybenzoate de méthyle : demande de brevet européen : EP 179 619.

2-bromométhyl-6-méthoxybenzoate d'éthyle : J. Org. Chem., 1983, 48, 3439-3444.

2-bromométhyl-4,5-diméthoxybenzoate d'éthyle et 2-bromométhyl-3,4-diméthoxybenzoate d'éthyle : J. Org. Chem., 1968, 33, 494.

4-bromométhyl-2-méthoxybenzoate de méthyle : Bull. Soc. Chim. France, 1962, 2255.

1-bromométhyl-4-cyano-2-méthoxybenzène : J. Med. Chem., 1990, 33, 2437-2451.

D'une manière générale, les dérivés halogénométhylbenzène peuvent être préparés par action du N-bromosuccinimide sur les dérivés de méthylbenzène correspondants. La réaction est effectuée dans un solvant comme le tétrachlorure de carbone en présence de péroxyde de dibenzoyle. On peut également préparer un dérivé d'halogénométhylbenzène à partir d'un dérivé d'hydroxyméthylbenzène correspondant par action du tribromure de phosphore dans l'éther.

Selon un autre procédé, les dérivés d'halogénométhylbenzène de formule (III) peuvent être préparés à partir de l'alcool correspondant par action du chlorure de thionyle pour préparer un chlorure de méthylbenzène.

Pour certaines valeurs des substituants $R_1$, $R_2$, $R_5$ et/ou $R_6$ les composés selon l'invention (I) peuvent être préparés à partir d'un précurseur de formule (I') substitué par un groupe $R'_1$, $R'_2$, $R'_5$ et/ou $R'_6$ appelé groupe précurseur de $R_1$, $R_2$, $R_5$ et/ou $R_6$ en utilisant des méthodes connues de l'homme de l'art.

Dans la description qui va suivre, on expose la préparation des composés de formule (I) portant des substituants $R_1$ et/ou $R_5$ ; les mêmes méthodes s'appliquent à la préparation des composés dans lesquels les substituants $R_2$ et/ou $R_5$ ont les valeurs indiquées pour $R_1$ et/ou $R_6$.

Les composés (I) dans lesquels $R_1$ et/ou $R_6$ est un hydroxy peuvent être obtenus par hydrogénation catalytique, par exemple en présence de palladium sur charbon, d'un composé de formule (I') dans lequel $R'_1$ et/ou $R'_6$ est un benzyloxy. Ces composés peuvent également être préparés à partir de composés analogues de formule (I') dans lesquels $R'_1$ et/ou $R'_6$ représente un groupe amino en utilisant la méthode décrite dans J. Org. Chem., 1977, 42, 2053.

Les composés de formule (I) dans lesquels $R_1$ et/ou $R_5$ représente un ($C_1$-$C_7$)alcoxy peuvent être préparés directement par le procédé selon l'invention à partir des composés de formule (II) et (III) correctement substitués.

Les composés (I') dans lesquels $R'_1$ et/ou $R'_6$ représente un hydroxy permettent également de préparer des composés (I) dans lesquels $R_1$ et/ou $R_6$ est un ($C_1$-$C_7$)alcoxy par action d'un halogénure d'alkyle en $C_1$-$C_7$ en présence d'une base telle qu'un hydrure métallique ou un carbonate alcalin ou alcalino-terreux comme $K_2CO_3$ ou $Cs_2CO_3$ dans un solvant tel que le THF ou le DMF. De même, on prépare les composés de formule (I) dans lesquels $R_1$ et/ou $R_6$ représente un ω-aminoalkyloxy par action d'une ω-chloroalkylamine sur les composés dans lesquels $R'_1$ et/ou $R'_6$ = OH ; de même on prépare les composés dans lesquels $R_1$ et/ou $R_6$ représente un ω-hydroxyalcoxy par action d'un chloroalkylalcool ; dans le cas particulier de la préparation d'un composé (I) dans lequel $R_1$ et/ou $R_6$ = $O(CH_2)_2OH$, on peut également faire agir le carbonate d'éthylène sur un composé (I') dans lequel $R'_1$ et/ou $R'_6$ = OH.

Les halogénures d'halogénoalcoxybenzyles (III, $R'_6$ = ω-halogénoalcoxy) sont utilisés pour la préparation de composés selon l'invention dans lesquels le substituant $R_6$ est un ω-aminoalcoxy non substitué ou substitué par un ou deux alkyles, selon le schéma suivant:

-O-Alk'-Hal + NHAA'-O-Alk'-NAA'

dans lequel Alk' représente un ($C_2$-$C_7$)alkyle, A et A' représentent indépendamment l'un de l'autre H ou un ($C_1$-$C_7$)alkyle.

Les composés de formule (I) dans lesquels $R_1$ et/ou $R_6$ représente un formyloxy ou respectivement un ($C_1$-$C_7$)alkylcarbonyloxy ou un benzoyloxy sont obtenus, par exemple par action de l'acide formique en présence de dicyclohexylcarbodiimide (J.HUANG et al. J.Chem. Research (S), 1991, 292-293) ou respectivement par action d'un halogénure d'acide ou d'un anhydride sur un composé (I') dans lequel $R'_1$ et/ou $R'_6$ est un hydroxy.

Les composés de formule (I) dans lesquel $R_6$ est un groupe $OR_7$, $R_7$ représentant un ω-carbamoyl ($C_1$-$C_7$)alkyle libre ou substitué par un ou deux alkyles en $C_1$-$C_7$ peuvent être préparés à partir d'un composé (I') dans lequel $R'_6$ représente un groupe $OR_7$, $R_7$ représentant un ω-carboxy($C_1$-$C_7$)alkyle estérifié par un alkyle en $C_1$-$C_7$. Cette préparation est effectuée de façon classique pour l'homme de l'art par action d'une amine correctement choisie.

Les composés de formule (I) dans lesquels $R_6$ représente un ($C_1$-$C_7$)alcoxycarbonyle peuvent se préparer directement par le procédé selon l'invention. Par des méthodes connues de l'homme de l'art, ils permettent d'obtenir les composés de formule (I) dans lesquels $R_6$ est un groupe carboxy.

Les composés de formule (I') dans lesquels $R'_6$ est un benzyloxycarbonyle permettent d'obtenir par hydrogénation catalytique les composés (I) dans lesquels $R_6$ est un carboxy. Par action d'un halogénure de thionyle, on obtient les composés de formule (I') dans lesquels $R'_6$ est un halogénocarbonyle. A partir de tels composés, on prépare des composés de formule (I) dans lesquels $R_6$ est un carbamoyle substitué par $R_{17}$ et $R_{18}$, par action d'un composé $HNR_{17}R_{18}$. On peut également utiliser les composés de formule (I') dans lesquels le substituant $R'_6$ est un phénoxycarbonyle pour obtenir les composés de formule (I) dans lesquels $R_6$ est un phénylcarbamoyle ou un ($C_1$-$C_7$)alkylcarbamoyle par action d'une aniline ou d'une ($C_1$-$C_7$)alkylamine. Une aniline substituée sur le phényle par l'un quelconque des substituants du groupe phényle définis dans Ar ou, respectivement, une alkylamine substituée sur l'alkyle par $R_{26}$ permettent d'obtenir des composés de formule (I) dans lesquels $R_6$ est un phénylcarbamoyle substitué sur le phényle par l'un quelconque des substituants du groupe phényle définis dans Ar ou, respectivement, un alkylcarbamoyle substitué sur l'alkyle par $R_{26}$.

On peut utiliser les composés de formule (I') dans lesquels $R'_6$ est un carboxy pour obtenir les composés de formule (I) dans lesquels $R_6$ est un groupe $-CONR_{17}R_{18}$ par action d'un composé de formule $HNR_{17}R_{18}$ en présence de BOP et d'une amine telle que la diisopropyléthylamine.

De la même façon, les composés de formule (I) dans lesquels $R_6$ est un groupe $CONHCR_{10}R_{23}COR_{12}$ sont préparés à partir de composés de formule (I') dans lesquels $R'_6$ représente soit un groupe COCl, soit un groupe phénoxycarbonyle, par action de $H_2NCR_{10}R_{23}COR_{12}$. Ils peuvent également être préparés à partir de composés de formule (I') dans lesquels $R'_6$ est un carboxy par réaction avec un composé $H_2NCR_{10}R_{23}COR_{12}$ en présence de BOP et d'une amine telle que la diisopropyléthylamine.

Les composés de formule (I) dans lesquels $R_6$ est un groupe $COR_{25}$ sont préparés à partir de composés (I') correspondants dans lesquels $R'_6$ est un phénoxycarbonyle, par action de $R_{25}H$.

On peut préparer un composé (I) dans lequel $R_6$ est un thiocarbamoyle par réaction du réactif de Lawesson sur un composé (I) dans lequel $R_6$ est le carbamoyle correspondant.

Un composé de formule (I') dans lequel $R'_6$ est un groupe nitro permet d'obtenir par hydrogénation catalytique, par exemple en présence d'oxyde de platine, de nickel de Raney® ou de palladium sur charbon, ou par réduction chimique, par exemple en présence d'étain ou de fer en milieu acide, un composé (I) dans lequel $R_6$ est un groupe amino ; on peut ensuite préparer d'autres composés dans lesquels le groupe amino est substitué en utilisant des réactions bien connues de l'homme de l'art.

Pour préparer des composés de formule (I) dans lesquels $R_1$ et/ou $R_6$ représente un ($C_1$-$C_7$)monoalkylamino, on fait réagir un composé de formule (I') dans lequel $R'_1$ et/ou $R'_6$ représente un groupe amino avec un

aldéhyde ou une cétone, en milieu acide, en présence d'un agent réducteur tel que le cyanoborohydrure de sodium ; par une réaction identique, on prépare les composés (I) dans lesquels $R_1$ et/ou $R_6$ représente un dialkylamino.

On peut préparer les composés de formule (I) dans lesquels $R_6$ représente un groupe amino substitué par un benzyle, lui-même éventuellement substitué, ou par un alcène en $C_3$-$C_8$, par action d'un chlorure de benzyle ou d'un chloroalcène en $C_3$-$C_8$ sur un composé de formule (I') dans lequel $R'_6$ est un groupe amino ou ($C_1$-$C_7$)alkylamino.

Les composés de formule (I) dans lesquels $R_6$ représente un groupe Δ3-pyrrolin-1-yle se préparent par action, sous atmosphère inerte, du cis-1,4-dichlorobut-2-ène sur les composés de formule (I') dans lesquels $R'_6$ est un groupe amino en présence d'une base, telle que la triéthylamine. Par hydrogénation on prépare ensuite les composés de formule (I) dans lesquels $R_6$ est un groupe pyrrolidin-1-yle.

La réaction du cis-1,4-dichlorobut-2-ène avec les composés (I') dans lesquels $R'_6$ est un groupe amino, peut également s'effectuer à l'air en présence d'une base, telle que le carbonate de sodium et conduit, dans ces conditions, à la formation d'un mélange d'un composé de formule (I) dans laquelle $R_6$ représente un groupe Δ3-pyrrolin-1-yle et d'un composé de formule (I) dans laquelle $R_6$ représente un groupe pyrrol-1-yle, que l'on peut séparer par chromatographie.

Les composés de formule (I) dans lesquels $R_6$ représente un groupe isoindolin-2-yle se préparent par action de l'α,α'-dibromo-o-xylène sur les composés de formule (I') dans lesquels $R'_6$ est un groupe amino, en présence d'une base telle que la triéthylamine, et dans un solvant tel que le diméthylformamide à reflux.

Les composés de formule (I) dans lesquels $R_6$ représente un groupe 1-méthyl-2,4-dioxoimidazolin-3-yle ($NR_6R_9$=N-méthylhydantoïne) se préparent en deux étapes : on fait réagir la sarcosine sur un composé de formule (I') dans lequel $R'_6$ représente un phénoxycarboxamido, en présence d'une base telle que la triéthylamine, pour obtenir un composé de formule (I') dans lequel $R'_6$ représente un N'-carboxyméthyl-N'-méthyluréido, puis par chauffage à 100°C, sous vide, le produit obtenu précédemment se cyclise.

De la même façon, on prépare un composé de formule I dans laquelle $R_6$ représente le groupe 2,4-dioxoimidazolin-3-yle ($NR_6R_9$=hydantoïne) par action de la glycine sur un composé de formule (I') tel que défini cidessus.

Lorsque $R'_1$ et/ou $R'_6$ représente un amino, on peut également effectuer une nitrosation, par exemple en présence d'acide nitreux ou de nitrite de sodium pour préparer un composé (I') dans lequel $R'_1$ et/ou $R'_6$ représente un sel de diazonium ; par des réactions connues de l'homme de l'art, on accède alors aux composés (I) selon l'invention dans lesquels $R_1$ et/ou $R_6$ est un cyano, un halogéno ou un alkylthio en $C_1$-$C_7$. Enfin par des réactions classiques à partir de composés (I') dans lesquels $R'_1$ et/ou $R'_6$ = $NH_2$, on peut préparer des composés (I) dans lesquels $R_1$ et/ou $R_6$ représente l'un des groupes de formule, RCONH-, ROCONH-, RNHCONH- ou $RSO_2NH$-, dans lesquels R représente un ($C_1$-$C_7$)alkyle, un groupe Ar ou un groupe $-CH_2$-Ar.

Les composés de formule (I) dans lesquels $R_6$ représente un groupe $-CH_2HN_2$ se préparent à partir de composés analogues de formule (I') dans lesquels $R'_6$ représente un groupe cyano selon la méthode décrite dans J. Med. Chem., 1990, 33, 2437-2451. A partir de ces composés, on prépare les composés de formule (I) dans lesquels $R_6$ représente un groupe $-CH_2NR_6R_9$, $R_9$ et/ou $R_8$ étant différent de l'hydrogène, selon des méthodes connues de l'homme de l'art.

Les composés (I) dans lesquels $R_6$ représente un groupe $-NR_8R_9$, $R_9$ étant un formyle, ou respectivement un ($C_1$-$C_7$)alkylcarbonyle, un ($C_3$-$C_7$)cycloalkylcarbonyle, un benzoyle éventuellement substitué, un pyridylcarbonyle, un méthylpyridylcarbonyle, un thiénylcarbonyle, s'obtiennent par action de l'acide formique en présence d'anhydride acétique sur un composé (I') dans lequel $R'_6$ est un groupe amino ou respectivement par action de l'anhydride approprié ou du chlorure d'acide approprié sur un composé (I') dans lequel $R'_6$ est un groupe amino, en présence d'une amine comme la triéthylamine.

De la même façon, on fait agir le chlorure d'acide $R_{11}R_{19}NCR_{10}R_{23}COCl$ sur un composé de formule (I') dans lequel $R'_6$ est un groupe amino pour préparer un composé de formule (I) dans lequel $R_6$ est un amino substitué par $COCR_{10}R_{23}NR_{11}R_{19}$.

Pour préparer un composé de formule (I) dans lequel $R_6$ représente un groupe $-NR_8CO$-($C_2$-$C_7$)alkyl-$NR_{11}R_{19}$, on fait réagir un halogénure d'halogéno($C_2$-$C_7$)alkylcarbonyle, tel que le chlorure de 3-chloropropionyle ou le chlorure de 4-chlorobutyryle par exemple, sur un composé de formule (I') dans lequel $R'_6$ est un groupe $-NHR_8$, en présence d'une base comme la triéthylamine ; puis par réaction du composé obtenu avec une amine $HNR_{11}R_{19}$ on obtient le composé de formule (I) désigné ci-dessus.

De même par action d'un halogénure d'ω-benzyloxy-($C_1$-$C_7$)alkylcarbonyle, sur un composé de formule (I') dans lequel $R'_6$ représente un groupe $-NHR_6$, on prépare un composé de formule (I) dans lequel $R_6$ représente un groupe $-NR_8CO$-($C_1$-$C_7$)alkyl-O-$CH_2$-$C_6H_5$. Par hydrogénation du composé précédent, en présence d'un catalyseur tel que le palladium sur charbon à 5 %, on obtient un composé de formule (I) dans lequel $R_6$ est un groupe $-NHR_8$-CO-($C_1$-$C_7$)alkyl-OH.

Selon un autre exemple de préparation, un composé (I) dans lequel $R_6$ est un groupe $(C_1-C_7)$alkylsulfonamido ou respectivement un benzylsulfonamido, un groupe $-NHSO_2Ar$ ou un groupe $-NHSO_2NR_{16}R_{22}$ s'obtient par action d'un halogénure de $(C_1-C_7)$alkylsulfonyle ou respectivement d'un halogénure de benzylsulfonyle, d'un composé $ArSO_2Cl$ ou d'un composé $R_{22}R_{16}NSO_2Cl$ sur un composé (I') dans lequel $R'_6$ est un groupe amino.

Les composés (I') dans lesquels $R'_6$ représente un groupe amino sont également utiles pour la préparation de composés dans lequel ce groupe amino est substitué par un groupe $(CH_2)_t-COR_{12}$. Dans ce cas, on fait agir sur (I') en présence de chlorure cuivreux un composé de formule $Hal-(CH_2)_t-COOAlk$ dans lequel Hal représente un halogène, par exemple le brome et Alk représente un alkyle en $C_1-C_7$ ; le cas échéant, on transforme l'ester ainsi obtenu en acide ou en amide. On peut utiliser l'action d'un anhydride, tel que l'anhydride succinique ou l'anhydride glutarique, sur un composé (I') dans lequel $R'_6$ est un amino pour préparer un composé (I) dans lequel $R_6$ est un groupe $-NHCO(CH_2)_2CO_2H$ ou $-NHCO(CH_2)_3CO_2H$. Le cas échéant on transforme l'acide ainsi obtenu en ester ou en amide.

On peut également utiliser l'action du chlorure d'éthyloxalyle ou respectivement du chlorure d'éthylmalonyle sur un composé (I') dans lequel $R'_6$ est un amino pour préparer un composé (I) dans lequel $R_6$ est un groupe $-NHCOCO_2Et$ ou respectivement un groupe $-NHCOCH_2CO_2Et$.

De la même façon, les composés de formule (I) dans lesquels $R_6$ est un groupe amino substitué par un groupe $CR_{10}R_{23}COR_{12}$ sont préparés par action d'un composé de formule $Hal-CR_{10}R_{23}COR_{12}$ sur les composés (I') correspondants dans lesquels le substituant $R'_6$ est un amino.

Par action d'un chloroformiate d'alkyle en $C_1-C_7$, de phényle ou de benzyle sur un composé (I') dont le substituant $R'_6$ est un amino, on prépare un composé (I) dans lequel $R_6$ est un groupe amino substitué par un alcoxycarbonyle, un phénoxycarbonyle ou un benzyloxycarbonyle.

De même, par action d'un chlorure de phénoxythiocarbonyle sur un composé de formule (I') dans lequel $R'_6$ est un groupe amino, on obtient un composé de formule (I) dans lequel $R_6$ est un phénoxythiocarbonylamino.

Par action de l'ammoniac sur un composé de formule (I') dans lequel $R'_6$ est un groupe amino substitué par un phénoxycarbonyle ou un phénoxythiocarbonyle, on prépare un composé de formule (I) dans lequel $R_6$ est un uréido ou un thiouréido ; par action sur un tel composé de formule (I') d'une aniline correctement substituée ou d'une mono ou dialkylamine en $C_1-C_7$ correctement substituées, on prépare un composé de formule (I) dans lequel $R_6$ est un N'-phényluréido correctement substitué, un N'-alkyluréido ou N',N'-dialkyluréido dans lesquels l'alkyle est en $C_1-C_7$ correctement substitués.

On peut également préparer d'autres composés (I) dans lequel $R_6$ est un uréido ($-NHCONR_{14}R_{24}$) ou respectivement, un thiouréido ($-NHCSNR_{14}R_{24}$) par action d'un composé $NHR_{14}R_{24}$, sur un composé (I') dans lequel $R'_6$ est un groupe phénoxycarbonylamino ou, respectivement, phénoxythiocarbonylamino.

On peut aussi préparer un composé (I) dans lequel $R_6$ est un uréido ($-NHCONR_{14}R_{24}$) ou respectivement un thiouréido par action d'un chlorure de carbamoyle ($ClCONR_{14}R_{24}$), ou respectivement d'un chlorure de thiocarbamoyle, sur un composé de formule (I') dans lequel $R'_6$ est un groupe amino.

On peut encore préparer un composé (I) dans lequel $R_6$ est un thiouréido par action du réactif de Lawesson sur un composé (I') dans lequel $R'_6$ est l'uréido correspondant.

Les composés (I) dans lesquels $R_6$ est un groupe guanidino non substitué ou substitué une ou deux fois par un alkyle en $C_1-C_7$, un phényle ou un benzyle peuvent être préparés à partir des composés (I') dans lesquels $R'_6$ est un groupe phénoxyamido par action du cyanamide ou d'un de ses dérivés correctement substitué sur l'azote.

Les composés (I) dans lesquels $R_6$ est un groupe guanidino substitué en position 2 par un cyano se préparent en deux étapes : on fait réagir le diméthyl N-cyanodithioiminocarbonate sur un composé (I') dans lequel $R'_6$ est un amino, dans un solvant tel que le n-butanol à reflux, pour obtenir un composé (I') dans lequel $R'_6$ est un groupe $-NHC(SCH_3)=N-CN$ ; par réaction du composé précédent avec une amine appropriée, on obtient le composé (I) attendu.

On peut également préparer un composé (I) dans lequel $R_6$ est un groupe amino substitué par un $(C_1-C_7)$alkylcarbamoyle ou par un phénylcarbamoyle par action d'un isocyanate d'alkyle ou de phényle sur un composé (I') dont le substituant $R'_6$ est un amino.

Par ailleurs, un composé (I) dans lequel $R_6$ est un groupe sulfamoyle substitué par $R_{20}$ et $R_{21}$, est préparé par action d'un composé $HNR_{20}R_{21}$ sur un composé de formule (I') dans lequel $R'_6$ est un groupe halogénosulfonyle.

L'affinité des composés selon l'invention pour les récepteurs de la vasopressine a été déterminée in vitro en utilisant la méthode décrite dans C. J. Lynch et al., J. Biol. Chem., 1985, 260 (5), 2844-2851. Cette méthode consiste à étudier le déplacement de la vasopressine tritiée fixée aux sites $V_1$ de membranes de foie de rats. Les concentrations inhibitrices de 50 % ($CI_{50}$) de la fixation de la vasopressine tritiée des composés selon

l'invention sont faibles, allant jusqu'à $10^{-7}$M.

L'affinité des composés (I) selon l'invention pour les récepteurs $V_2$ a été mesurée sur une préparation membranaire de rein de boeuf selon une méthode adaptée de P. Crause et al., Molecular and Cellular Endocrinology, 1982, 28, 529-541 et de F.L. Stassen et al., J. Pharmacol. Exp. Ther., 1982, 223, 50-54. Les composés selon l'invention inhibent la fixation de l'arginine-vasopressine tritiée aux récepteurs de la préparation membranaire. Les $CI_{50}$ des composés selon l'invention sont faibles, allant jusqu'à $10^{-8}$M.

L'activité antagoniste des récepteurs $V_2$ des composés selon l'invention a été montrée par le test de dosage de l'activité adénylate cyclase effectué selon une méthode adaptée de M. Laburthe et al., Molecular Pharmacol., 1986, 29, 23-27. On utilise une préparation membranaire de rein de boeuf et chaque produit est incubé 10 minutes à 37°C, seul ou en présence d'AVP (arginine vasopressine) à la concentration de $3.10^{-8}$M. L'AMP cyclique (adénosine monophosphate cyclique) produite est mesurée par dosage radioimmunologique. On détermine la concentration inhibant de 50 %($CI_{50}$) la stimulation de l'adénylate cyclase induite par $3.10^{-8}$M d'AVP. Les $CI_{50}$ déterminées sont de l'ordre de $10^{-7}$M, allant jusqu'à $10^{-8}$M.

L'activité agoniste ou antagoniste des récepteurs de la vasopressine des composés selon l'invention, administrés par voie orale, est évaluée chez le rat (Souche OFA, Sprague-Dawley) en surcharge hydrique, traité à la vasopressine. L'activité antagoniste des composés, selon l'invention a été également évaluée chez le rate normalement hydraté (souche OFA ou souche Sprague-Dawley) selon la technique décrite dans Br. J. Pharmacol., 1992, 105, 787-791. L'effet diurétique a été observé pour certains composés à la dose de 10 mg/kg.

De même, l'affinité des composés (I) selon l'invention pour les récepteurs de l'ocytocine a été déterminée in vitro par déplacement d'un analogue radioiodé de l'ocytocine fixé aux récepteurs d'une préparation membranaire de glandes mammaires de rattes gestantes, selon une technique proche de celle décrite par J. Eland et al. dans Eur. J. Pharmacol., 1987, 147, 197-207. Les $CI_{50}$ des composés selon l'invention atteignent $10^{-7}$M.

Les composés selon l'invention sont actifs après administration par différentes voies, notamment par voie orale.

Aucun signe de toxicité n'est observé avec ces composés aux doses pharmacologiquement actives.

Ainsi les composés selon l'invention peuvent être utilisés dans le traitement ou la prévention de différentes affections vasopressine-dépendantes ou ocytocine dépendantes, les affections cardiovasculaires, comme l'hypertension, l'hypertension pulmonaire, l'insuffisance cardiaque, l'infarctus du myocarde, ou le vasospasme coronaire, en particulier chez le fumeur, les angines instables et PTCA (percutaneous transluminal coronary angioplasty), l'ischémie cardiaque, les dérèglements de l'hémostase notamment l'hémophilie, le syndrome de Von Willebrand ; les affections du système nerveux central, la migraine, le vasospasme cérébral, l'hémorragie cérébrale, les oedèmes cérébraux, la dépression, l'anxiété, les états psychotiques, les troubles de la mémoire par exemple ; les affections du système rénal comme les oedèmes, le vasospasme rénal, la nécrose du cortex rénal, l'hyponatriémie, l'hypokaliémie, le syndrome de Schwartz Bartter ; les affections du système gastrique comme le vasospasme gastrique, l'hépatocirrhose, les ulcères, la pathologie des vomissements, par exemple la nausée y compris la nausée due à une chimiothérapie, le mal des transports, ou encore le syndrome de la sécrétion inappropriée de l'hormone antidiurétique (SIADH), le diabète insipide et l'énurésie. Les composés selon l'invention peuvent également être utilisés dans le traitement des troubles du comportement sexuel ; chez la femme, les composés selon l'invention peuvent être utilisés pour traiter la dysménorrhée ou le travail prématuré. On peut également utiliser les composés selon l'invention dans le traitement des cancers pulmonaires à petites cellules, des encéphalopathies hyponatriémique, de la maladie de Raynaud, le syndrome pulmonaire, le glaucome et dans les traitements post-opératoires, notamment après une chirurgie abdominale.

La présente invention a également pour objet des compositions pharmaceutiques contenant une dose efficace d'un composé selon l'invention ou d'un sel pharmaceutiquement acceptable et des excipients convenables.

Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intra-veineuse, topique, intratrachéale, intranasale, transdermique ou rectale, les principes actifs de formule (I) ci-dessus, ou leurs sels éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule (I) ci-dessus ou d'un des sels pharmaceutiquement acceptables, d'autres principes actifs qui peuvent être utiles dans le traitement des troubles ou maladies indiquées ci-dessus.

Ainsi, la présente invention a également pour objet des compositions pharmaceutiques contenant plusieurs principes actifs en association dont l'un est un composé selon l'invention.

Ainsi, selon la présente invention, on peut préparer des compositions pharmaceutiques contenant un composé selon l'invention associé à un composé agissant sur le système rénine-angiotensine tel qu' un inhibiteur de l'enzyme de conversion, un antagoniste de l'angiotensine II, un inhibiteur de la rénine. On peut également associer un composé selon l'invention, par exemple, avec un vasodilatateur périphérique, un inhibiteur calcique, un beta-bloquant, un alpha-1-bloquant ou un diurétique. De telles compositions seront utiles en particulier dans le traitement de l'hypertension ou de la défaillance cardiaque.

On peut également associer deux composés selon l'invention : un antagoniste spécifique du récepteur $V_1$ à un antagoniste spécifique du récepteur $V_2$ ou bien un antagoniste spécifique du récepteur $V_1$ à un antagoniste spécifique de l'ocytocine.

Ces associations permettront de renforcer les activités thérapeutiques des composés selon l'invention.

Préparation des 1,3-dihydro-indol-2-one

Préparation 1 :

1,3-Dihydro-4,6-diméthyl-3-spirocyclohexane indol-2-one.

Ce composé est préparé selon Moore et Plant dans J. Chem. Soc., 1951, 3475.

On maintient à reflux sous atmosphère inerte un mélange contenant 15 ml de quinoléine et 10 g d'oxyde de calcium et l'on ajoute en 30 minutes 5 g de 3,5-diméthylphénylhydrazide de l'acide cyclohexane carboxylique (IV, $R'_1$, $R'_2$ = $CH_3$, $CR_3R_4$ = cyclohexane). Le milieu réactionnel est refroidi puis versé dans un mélange glace-acide chlorhydrique. On extrait par de l'acétate d'éthyle, lave par de l'acide chlorhydrique 1N, par de l'eau jusqu'à neutralité, puis on sèche sur $Na_2SO_4$ et concentre sous vide pour obtenir un solide brun. Par trituration dans l'éther iso, on obtient le composé attendu. F = 223°C

En opérant de la même façon et en faisant varier l'hydrazide de départ, on obtient les dérivés 1,3-dihydroindol-2-one décrits dans le tableau 1 ci-dessous.

Ces composés sont purifiés par chromatographie sur colonne de silice, en éluant par du DCM ou par chromatographie sur colonne d'alumine en éluant par du DCM ou par de l'éther iso.

TABLEAU 1

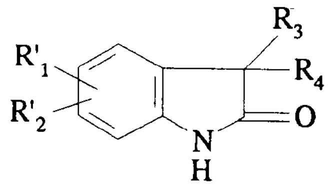

| R'1 | R'2 | CR3R4 | F °C |
|---|---|---|---|
| Cl-5 | H | cyclobutane | 191 |
| Cl-5 | H | cyclopentane | 189 |
| Cl-5 | H | cyclohexane | 186 |
| H | H | cyclohexane | 123-124 |
| $CH_3$-5 | H | cyclohexane | 164 |
| $CH_3O$-5 | H | cyclohexane | 226 |
| Cl-6 | H | cyclohexane | 168 |
| $CF_3O$-5 | H | cyclohexane | 164 |
| $C_6H_5O$-5 | H | cyclohexane | 160 |

Préparation 2 :

La 1,3-dihydro-3-spirocyclohexane indol-2-one décrite dans le tableau 1 ci-dessus peut également être obtenue par alkylation de l'indol-2-one en utilisant le procédé selon A.S. Kende et J.C. Hodges ou une variante décrite ci-après.

On maintient à -40°C, sous atmosphère d'azote, une solution de 30 g de 1,3-dihydro-indol-2-one dans 900 ml de THF et l'on ajoute 101 g de *tert*-butylate de potassium. On laisse remonter la température à 0°C en 1 heure, puis on refroidit à -60°C et l'on ajoute goutte à goutte une solution de 52 g de 1,5-dibromopentane dans 50 ml de THF. Après 30 minutes à -60°C, on laisse remonter à TA, puis on ajoute 30 ml d'eau et évapore le solvant sous pression réduite. Le résidu est repris par 500 ml de DCM et 200 ml d'eau puis on filtre l'insoluble et sépare la phase organique qui est lavée avec 100 ml d'eau, séchée sur sulfate de magnésium et évaporée sous vide. Le résidu est chromatographié sur silice en éluant par un mélange cyclohexane-éther. On obtient le composé attendu qui est recristallisé dans l'heptane. m = 34 g, F = 123-124°C.

A partir d'autres 1,3-dihydro-indol-2-ones et d'autres agents alkylants, on peut appliquer un mode opératoire semblable.

A titre d'exemple, parmi les composés de départ de formule (VII), la 5-chloro-1,3-dihydro-indol-2-one est décrite par Wright dans J. Am. Chem. Soc., 1956, 78, 221 et par Rajanbabu dans J. Org. Chem., 1986, 51, 1704. La 4-chloro-1,3-dihydroindol-2-one peut être préparée à partir du 2-chloro-6-nitrotoluène, selon la méthode décrite dans J. Am. Chem. Soc., 1956, 78, 221.

La 1,3-dihydro-5-méthoxyindol-2-one est préparée à partir de la 4-méthoxyaniline selon la méthode décrite dans J. Am. Chem. Soc., 1974, 96, 5512. De la même manière à partir du dérivé aniline approprié, on prépare diverses 1,3-dihydroindol-2-one :

Préparation 3

5-Ethoxy-1,3-dihydro-indol-2-one :

A-3-Méthylthio-5-éthoxy-1,3-dihydro-indol-2-one.

A une solution refroidie vers -70°C de 12,5 g de chlore dans 400 ml de DCM on ajoute 23,6 g de méthylthio acétate d'éthyle dans 60 ml de DCM. Après 5 minutes d'agitation à cette même température on additionne une solution de 4-éthoxyaniline (48,3 g) dans 120 ml de DCM. On agite une heure vers -70°C, ajoute 39,3 ml de triéthylamine et laisse remonter à température ambiante. On ajoute 200 ml d'eau, décante la phase organique qui est séchée sur sulfate de magnésium et évaporée sous pression réduite. Le résidu est repris dans 500 ml d'isopropanol et 20 ml d'acide chlorhydrique concentré. On agite environ 16 heures à température ambiante, filtre et sépare le précipité. Le filtrat est concentré sous pression réduite pour donner le produit attendu.

B - 5-Ethoxy-1,3-dihydro-indol-2-one :

Le solide précédent dans 1500 ml d'isopropanol est déthiométhylé en présence de 100 g de nickel de Raney® (80 à 100 $m^2$ par g)à reflux durant 3 heures sous atmosphère d'azote. On filtre sur talc, rince avec 1000 ml d'isopropanol et concentre le filtrat sous pression réduite. On isole après recristallisation dans le toluène 16 g du produit attendu. F= 156°C

De la même manière, à partir des anilines correspondantes on isole :

5-benzyloxy-1,3-dihydro-indol-2-one F = 152°C
5-n-propyl-1,3-dihydro-indol-2-one F = 136°C
5-éthyl-1,3-dihydro-indol-2-one F = 152°C
5-(2,2,2-trifluoroéthoxy)-1,3-dihydro-indol-2-one F = 145°C
5-trifluorométhyl-1,3-dihydro-indol-2-one F = 193°C
5-fluoro-1,3-dihydro-indol-2-one F = 143°C.

En opérant selon la technique décrite dans la préparation 2 et en faisant varier le dérivé 1,3-dihydro-indol-2-one de départ et le réactif alkylant, on obtient les composés de formule (II) décrits ci-dessous.

TABLEAU 2

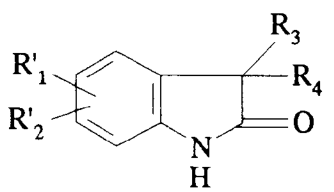

(II)

| $R'_1$ | $R'_2$ | $CR_3R_4$ | F °C | Réactif alkylant |
|---|---|---|---|---|
| Cl-5 | H | cyclohexane | 186-189 | $Br(CH_2)_5Br$ |
| Cl-5 | H | cycloheptane | 202 | $Br(CH_2)_6Br$ |

TABLEAU 2 (suite)

| $R'_1$ | $R'_2$ | $CR_3R_4$ | F °C | Réactif alkylant |
|---|---|---|---|---|
| Cl-5 | H | 4,4-diméthyl-cyclohexane | 180 | $TsO(CH_2)_2C(CH_3)_2-$ $-(CH_2)_2OTs$ |
| Cl-5 | H | hexahydroindane-2 | 223 | cis-diiodométhyl-1,2 cyclohexane |
| $CH_3O$-5 | H | 4,4-diméthyl-cyclohexane | 202 | $TsO\ (CH_2)_2C(CH_3)_2-$ $-(CH_2)_2-OTs$ |
| Cl-5 | H | indane-2 | 228 | a,a'-dibromométhyl-orthoxylène |
| Cl-5 | H | N-méthyl-pipéridine-4 | 260 | $Cl(CH_2)_2N(CH_3)-$ $-(CH_2)_2Cl$ |
| Cl-5 | H | tétrahydropyranne-4 | 223 | $I(CH_2)_2O(CH_2)_2I$ |
| Cl-4 | H | cyclohexane | 215 | $Br(CH_2)_5Br$ |
| BzO-5 | H | cyclohexane | 162 | $Br(CH_2)_5Br$ |
| Cl-5 | H | 2,3-dihydro-phénalène-2 | - | 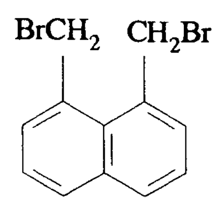 |
| BzO-5 | H | 4,4-diméthyl-cyclohexane | 154 | $TsO(CH_2)_2C(CH_3)_2-$ $-(CH_2)_2OTs$ |
| Cl-5 | H | 4-spirocyclopentane cyclohexane | 202 | 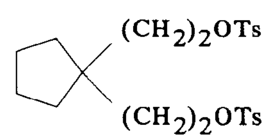 |
| nPr-5 | H | cyclohexane | 151 | $Br(CH_2)_5Br$ |

TABLEAU 2 (suite)

| $R'_1$ | $R'_2$ | $CR_3R_4$ | F °C | Réactif alkylant |
|---|---|---|---|---|
| EtO-5 | H | N-tBu-pipéridine-4 | - | 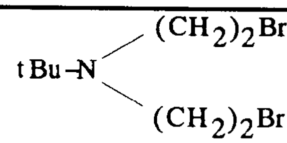 |
| Cl-5 | H | N-Bz-pipéridine-4 | 165 | 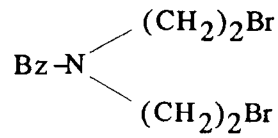 |
| Cl-5 | H | N-phényl-pipéridine-4 | 188 | 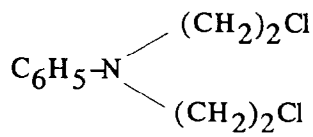 |
| Cl-5 | H | 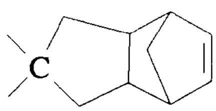 | 300 | 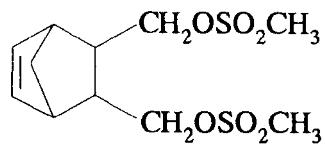 |
| EtO-5 | H | 4,4-diéthyl-cyclohexane | 132 | $TsO(CH_2)_2C(C_2H_5)_2$ $-(CH_2)_2OTs$ |
| EtO-5 | H | cyclohexane | 163 | $Br(CH_2)_5Br$ |
| EtO-5 | H | 4,4-diméthyl-cyclohexane | 178 | $TsO(CH_2)_2C(CH_3)_2-$ $-(CH_2)_2OTs$ |
| EtO-5 | H | cycloheptane | 139 | $Br(CH_2)_6Br$ |
| Et-5 | H | 4,4-diméthyl-cyclohexane | 160 | $TsO(CH_2)_2C(CH_3)_2-$ $-(CH_2)_2OTs$ |
| $CF_3CH_2O$ -5 | H | 4,4-diméthyl-cyclohexane | 164 | idem |
| H | H | 4,4-diméthyl-cyclohexane | 169 | idem |

TABLEAU 2 (suite)

| R'1 | R'2 | $CR_3R_4$ | F °C | Réactif alkylant |
|---|---|---|---|---|
| $CF_3$-5 | H | 4,4-diméthyl-cyclohexane | 211 | idem |
| F-5 | H | 4,4-diméthyl-cyclohexane | 171 | idem |
| Cl-5 | H | 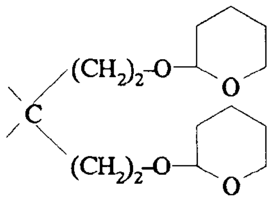 | 120 | 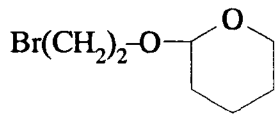 |
| EtO-5 | H | 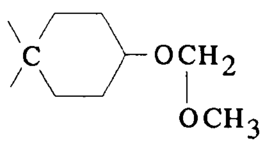 | RMN (1) | 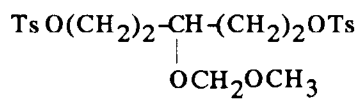 |
| EtO-5 | H | 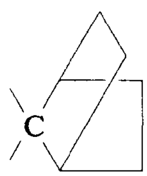 | 208 | 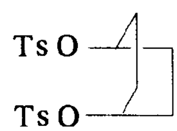 |
| EtO-5 | H | 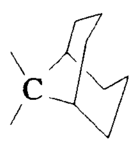 | 214 | 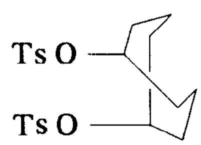 |
| EtO-5 | H | tétrahydro-pyrane-4 | 146 | $I(CH_2)_2O(CH_2)_2I$ |
| EtO-5 | H | 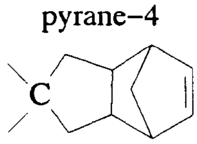 | 255 | 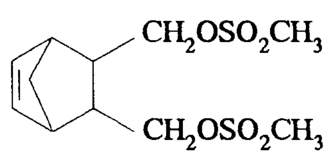 |

(1) RMN à 200 MHz dans $CDCl_3$ :

8,3 ppm : s : 1 H

7,1 ppm : d : 1 H

6,7 ppm : m : 2 H

4,7 ppm : s : 2 H

3,9 ppm : q : 2 H
3,8 ppm : m : 1 H
3,4 ppm : s : 3 H
2,2 ppm : m : 2 H
1,8 ppm : m 6 H
1,4 ppm : t : 3 H

Préparation 4 :

1,3-Dihydro-3-spiroadamantaneindol-2-one.

Ce composé est préparé selon I. Fleming et al., Tetrahedron Letters, 1982, 2053-2056 à partir de 2-bromoaniline et d'adamantane-2-one.

Préparation 5 :

1,3-Dihydro-5-nitro-3-spirocyclohexaneindol-2-one.

Ce composé est préparé selon la méthode décrite dans J. Am. Chem. Soc., 1945, 67, 499 par nitration de la 1,3-dihydro-3-spirocyclohexaneindol-2-one. F = 192°C.

De la même manière, à partir de 1,3-dihydro-3-spiroadamantaneindol-2-one, on prépare la 1,3-dihydro-5-nitro-3-spiroadamantaneindol-2-one. F > 260°C

On prépare également la 1,3-dihydro-5-nitro-3-spiro(4,4-diméthyl) cyclohexane)indol-2-one. F = 195°C

Préparation 6 :

5-amino-1,3-dihydro-3-spirocyclohexaneindol-2-one.

Ce composé est préparé selon la méthode décrite dans J. Chem. Soc., 1951, 3475, par réduction de la 1,3-dihydro-5-nitro-3-spirocyclohexaneindol-2-one, préparée précédemment. F = 176°C.

De la même manière, on prépare la 5-amino-1,3-dihydro-3-spiroadamantaneindol-2-one, F = 245°C

Préparation 7 :

5-fluoro-1,3-dihydro-3-spirocyclohexaneindol-2-one.

A - tétrafluoroborate de 5-diazonium-1,3-dihydro-3-spirocyclohexaneindol-2-one.

On refroidit à 0°C, une solution contenant 4 g de 5-amino-1,3-dihydro-3-spirocyclohexaneindol-2-one dans 9,2 ml d'acide chlorhydrique 6N et l'on ajoute 2,27 g de nitrite de sodium dans 2,6 ml d'eau puis 2,54 g de tétrafluoroborate de sodium dans 9 ml d'eau. Après 5 minutes sous agitation, on filtre le précipité, lave avec une solution de tétrafluoroborate à 5 %, avec 3 ml de méthanol refroidi vers 0°C, puis avec 5 ml d'éther. Le sel obtenu est séché sous vide à TA en présence d'anhydride phosphorique.

B - 5-fluoro-1,3-dihydro-3-spirocyclohexaneindol-2-one.

1 g du composé obtenu à l'étape A est placé dans 5 ml de xylène et chauffé vers 115°C pendant 2 heures. On refroidit à TA, filtre le précipité, rince au toluène et ajoute au filtrat 0,1 g de charbon actif. Après filtration, le solvant est évaporé sous pression réduite et l'on obtient 0,45 g du composé attendu que l'on recristallise dans du pentane. F = 114°C.

Préparation 8 :

5-cyano-1,3-dihydro-3-spirocyclohexaneindol-2-one.

On dissout à TA 4,78 g de cyanure de potassium et 4,95 g de cyanure cuivreux dans 40 ml de DMSO. On refroidit vers 15°C et l'on ajoute 4,15 g du sel de diazonium obtenu dans la préparation précédente à l'étape A.

Après 30 minutes d'agitation à TA, on ajoute 100 ml d'eau et 100 ml d'éther puis on sépare la phase organique qui est séchée sur sulfate de magnésium et évaporée sous pression réduite. Le résidu est chromatographié sur silice en éluant avec un mélange cyclohexane-éther. On obtient le composé attendu qui est recristallisé dans l'heptane. m = 1,4 g, F = 216°C.

Préparation 9 :

5-chloro-1,3-dihydro-3-spiroadamantaneindol-2-one.

On dissout 1 g de p-chlorophénylhydrazide de l'acide adamantane-2-carboxylique dans du THF et on ajoute, à -40°C, 2,5 ml de n-butyllithium en solution (1,6 M dans l'hexane). Après 5 minutes d'agitation, on concentre sous vide en maintenant à une température inférieure à 30°C. On ajoute 30 ml de 1,2,3,4-tétraméthybenzène et l'on chauffe à reflux pendant 1 heure. On concentre sous pression réduite, reprend le résidu par de l'acide chlorhydrique normal, extrait à l'éther, lave, sèche et concentre sous vide. L'huile obtenue est chromatographiée sur colonne de silice en éluant par DCM ; on obtient 0,3 g du produit attendu sous forme d'une cire que l'on cristallise dans l'éther iso. F = 249°C.

Préparation 10 :

5-Acétyl-1,3-dihydro-3-spirocyclohexaneindol-2-one.

A une solution refroidie à 5°C de 4 g de 1,3-dihydro-3-spirocyclohexaneindol-2-one dans 35 ml de 1,2-dichloroéthane, on ajoute 2,56 g de chlorure d'acétyle puis 8,25 g de chlorure d'aluminium anhydre. On chauffe 2 heures à reflux, évapore le solvant sous pression réduite, hydrolyse le milieu avec 50 g de glace et extrait à l'acétate d'éthyle.

La phase organique est lavée à l'eau, séchée sur sulfate de magnésium puis évaporée sous pression réduite. On chromatographie le résidu sur une colonne de silice en éluant avec un mélange d'heptane et d'éther éthylique et obtient 3,6 g du produit attendu. F = 192°C.

Préparation 11 :

5-Chloro-1,3-dihydro-3-spiro(4-tétrahydrothiopyrane)indol-2-one.

A. 5-Chloro-1,3-dihydro-3,3-di-(2-bromoéthyl)indol-2-one.

On ajoute lentement 7,66 g de brome dans un mélange refroidi vers 0°C de 12,4 g de triphénylphosphine dans 70 ml de DCM puis on ajoute 4,58 g de 5-chloro-1,3-dihydro-3,3-di[2-(tétrahydropyran-2-yloxy)éthyl]indol-2-one décrit dans le tableau 2. Après 16 heures à TA on ajoute 60 ml d'eau, sépare la phase organique qui est lavée avec 60 ml d'eau puis séchée sur sulfate de magnésium et évaporée sous vide. Le résidu est chromatographié sur une colonne de silice en éluant au DCM. On obtient 3,12 g du produit attendu. F = 215°C.

B. 5-Chloro-1,3-dihydro-3-spiro(4-tétrahydrothiopyrane)indol-2-one.

Sous atmosphère inerte, on ajoute 3 g du produit préparé à l'étape A dans 3,2 ml de DMF, 2 g de sulfure de sodium monohydrate et chauffe 2 heures à 50°C. On refroidit à TA, ajoute 6 ml d'eau et extrait au DCM. La phase organique est lavée à l'eau, séchée sur sulfate de magnésium et évaporée sous pression réduite. Le résidu huileux obtenu est purifié par chromatographie sur silice en éluant au DCM. On obtient 2,02 g du composé attendu.

Spectre RMN à 200 MHz dans $CDCl_3$.

8,12 ppm : s : 1 H
7,2 ppm : m : 2 H
6,8 ppm : d : 1 H
3,25 ppm : m : 2 H
2,65 ppm : m : 2 H
2 ppm : m : 4 H

Préparation 12 :

5-Ethoxy-1,3-dihydro-3-spiro[4-tricyclo[5.2.1.$0^{2,6}$]décane]indol-2-one.

On hydrogène pendant 16 heures, à 40°C et sous une pression de 20 bars, un mélange de 3 g de 5-éthoxy-1,3-dihydro-3-spiro[4-tricyclo[5.2.1.$0^{2,6}$]déc-8-ène]indol-2-one décrit dans le tableau 2, 1,5 g de palladium sur charbon à 5 % dans 160 ml de MeOH. On filtre le catalyseur sur Célite®, lave au MeOH et évapore sous vide le filtrat. On obtient 2,95 g du produit attendu, F = 236°C.

Préparations 13 et 14 :

5-Ethoxy-1,3-dihydro-3-spiro(4-méthoxycyclohexane)indol-2-one, isomère A et isomère B.

A) 3-Méthoxypentane-1,5-diol

A une solution de 30 g de 3-hydroxyglutarate de diéthyle, 33 ml de 2,6-di-*tert*-butylpyridine dans 500 ml de DCM, on ajoute 25 ml de trifluorométhylsulfonate de méthyle et chauffe à reflux pendant 5 heures. Après refroidissement, on ajoute 500 ml d'une solution d'HCl 0,5 N, décante la phase organique, sèche sur sulfate de magnésium et évapore sous vide le solvant. On reprend le solide obtenu dans 200 ml de THF anhydre, filtre puis refroidit le filtrat à -5°C. On ajoute alors lentement 160 ml d'une solution d'hydrure d'aluminium et de lithium 1M dans le THF et laisse 16 heures sous agitation en laissant remonter la température à TA. On refroidit le mélange réactionnel à 0°C et ajoute successivement 5,5 ml d'eau, 18 ml d'une solution à 15 % de NaOH et 5,5 ml d'eau. On filtre les sels minéraux et évapore sous vide le filtrat. On obtient le produit attendu après distillation sous pression réduite.

Eb = 104°C sous 1,5 Pa.

B) 3-Méthoxy-1,5-ditosyloxypentane

On refroidit à 0°C une solution de 31 g de chlorure de p-toluènesulfonyle, 26 ml de triéthylamine dans 120 ml de THF, ajoute 10 g du composé obtenu à l'étape précédente et laisse 24 heures sous agitation à TA. On ajoute 120 ml d'eau au mélange réactionnel, évapore sous vide le solvant, extrait à l'AcOEt, sèche sur sulfate de magnésium et évapore sous vide le solvant. On reprend l'huile obtenue dans 200 ml d'éther et 200 ml de NaOH 2N et laisse 16 heures sous agitation à TA. Après décantation, on sèche la phase organique sur sulfate de magnésium et évapore sous vide le solvant. On obtient 26 g du produit attendu après cristallisation dans le cyclohexane, F = 58°C.

C) 5-Ethoxy-1,3-dihydro-3-spiro(4-méthoxycyclohexane)indol-2-one, isomère A et isomère B.

On prépare ces composés selon le mode opératoire décrit à la Préparation 2 à partir de 11,85 g de 5-éthoxy-1,3-dihydroindol-2-one, 34 g de *tert*-butylate de potassium et 26 g du composé obtenu à l'étape précédente. On chromatographie sur silice en éluant par le mélange cyclohexane/AcOEt (80/20 ; v/v). On sépare les deux isomères :

- le moins polaire, isomère A : composé de la Préparation 13, F = 173°C ;
- le plus polaire, isomère B : composé de la Préparation 14, F = 186°C.

EXEMPLE 1

5-Chloro-1,3-dihydro-1-(3,4-diméthoxybenzyl)-3-spirocyclopentaneindol-2-one.

A une solution de 440 mg de 5-chloro-1,3-dihydro-3-spirocyclopentaneindol-2-one dans 5 ml de DMF, on ajoute par portions 66 mg d'hydrure de sodium à 80 % dans l'huile ; après 30 minutes d'agitation on introduit 510 mg de 1-bromométhyl-3,4-diméthoxybenzène puis on laisse 24 heures à TA sous agitation. On évapore le solvant sous vide puis on extrait à l'acétate d'éthyle, lave à l'eau et sèche sur sulfate de sodium. Après évaporation des solvants, le résidu est chromatographié sur silice en éluant par un mélange DCM/AcOEt (97/3, v/v). On obtient 250 mg du produit attendu.

Spectre RMN à 200 MHz dans le DMSO.

1,7-2,1 ppm : m : 8 H
3,7 ppm : s : 6 H
4,8 ppm : s : 2 H
6,7 ppm : d de d : 1 H
6,9 ppm : m : 3 H
7,2 ppm : d de d : 1 H
7,4 ppm : d : 1 H

EXEMPLE 2

1,3-Dihydro-1-(2,4-diméthoxybenzyl)-3-spiro(N-méthylpipéridine-4)indol-2-one.

On refroidit à -10° C sous azote, une solution de 2,08 g de 1-hydroxyméthyl-2,4-diméthoxybenzène dans 20 ml d'éther et on ajoute goutte à goutte une solution de 0,4 ml de tribromure de phosphore dans 8 ml d'éther ; le 1-bromométhyl-2,4-diméthoxybenzène ainsi obtenu est conservé à -30°C.

On refroidit à -50°C sous azote une solution de 1,78 g de 1,3-dihydro-3-spiro(N-méthylpipéridine-4)indol-2-one dans 170 ml de THF et on ajoute 0,970 g de *tert*-butylate de potassium et on laisse revenir à 0°C ; on refroidit à nouveau à -50°C et rajoute 1,380 g de *tert*-butylate de potassium. Après refroidissement à -70°C, on ajoute le dérivé bromé préparé ci-dessus puis on laisse remonter à TA. On ajoute 20 ml d'eau, concentre les solvants sous vide, extrait par AcOEt, sèche sur sulfate de sodium et évapore le solvant. Le résidu est chromatographié sur silice en éluant par un mélange DCM/MeOH (9/1, v/v). Le produit attendu cristallise dans le pentane, m = 1,260 g, F = 101°C.

EXEMPLE 3

1,3-Dihydro-1-(2,4-diméthoxybenzyl)-3-spiro(N-éthoxycarbonylpipéridine-4)indol-2-one.

On porte à reflux une solution de 1,152 g de chloroformiate d'éthyle dans 1,5 ml de benzène et on ajoute goutte à goutte une solution de 1,3 g du composé obtenu à l'exemple précédent dans 10 ml de benzène. Après 6 heures de chauffage à reflux, on évapore sous vide le solvant et reprend par de l'eau. On extrait à l'acétate d'éthyle, lave par une solution d'acide chlorhydrique 2N puis par de l'eau. Le produit attendu cristallise dans l'éther iso, m = 1,12 g, F = 156°C.

EXEMPLE 4

5-Chloro-1,3-dihydro-1-(3,5-diméthoxybenzyl)-3-spirocyclohexaneindol-2-one.

On ajoute par portions 0,03 g d'hydrure de sodium à 80 % dans l'huile à une solution de 0,236 g de 5-chloro-1,3-dihydro-3-spirocyclohexaneindol-2-one dans 5 ml de THF et on laisse 30 minutes sous agitation. On refroidit dans un bain de glace et ajoute lentement 0,190 g de 1-chlorométhyl-3,5-diméthoxybenzène puis on laisse revenir à TA. Après 5 heures de chauffage à reflux, on évapore le solvant sous vide et reprend par de l'eau. On extrait à l'éther, lave à l'eau, sèche sur sulfate de sodium et évapore les solvants puis on effectue une chromatographie sur silice en éluant par DCM/AcOEt (99/1, v/v). Le produit attendu cristallise dans l'éther iso, m = 0,044g, F = 106°C.

EXEMPLE 5

4-(5-Ethoxy-2,3-dihydro-2-oxo-3-spirocyclohexaneindol-1-yl)méthyl-3-méthoxybenzoate de méthyle.

A une solution de 5-éthoxy-1,3-dihydro-3-spirocyclohexaneindol-2-one dans 20 ml de DMF, on ajoute 0,33 g d'hydrure de sodium à 80 % dans l'huile, puis on introduit 2,85 g de 4-bromométhyl-3-méthoxybenzoate de méthyle. Après 4 heures sous agitation à TA, on évapore le solvant et reprend par de l'eau. On extrait au DCM, lave à l'eau, sèche sur sulfate de sodium et évapore les solvants, puis on chromatographie sur silice en éluant par un mélange DCM/AcOEt (97,5/2,5, v/v). Le produit attendu cristallise dans l'heptane, m = 3,445 g, F = 142°C.

EXEMPLE 6

Acide 4-(5-éthoxy-2,3-dihydro-2-oxo-3-spirocyclohexaneindol-1-yl)méthyl -3-méthoxybenzoïque

On laisse sous agitation pendant 4 heures à TA, une solution contenant 847 mg du composé préparé à l'exemple précédent et 420 mg de monohydrate d'hydroxyde de lithium dans 9 ml de MeOH, 9 ml de THF et 3 ml d'eau. On dilue par de l'eau, acidifie à pH 1 par addition d'acide chlorhydrique 1N, extrait au DCM, lave à l'eau.et sèche sur sulfate de sodium. Après évaporation du solvant, le produit attendu cristallise dans l'éther, m = 745 mg, F = 215-217°C.

EXEMPLE 7

5-Ethoxy-1,3-dihydro-1-[2-méthoxy-4-[N-(2-méthylphényl)carbamoyl] benzyl]-3-spirocyclohexaneindol-2-one.

A) Chlorure de l'acide 4-(5-éthoxy-2,3-dihydro-2-oxo-3-spirocyclohexaneindol-1-yl)méthyl-3-méthoxybenzoïque

On chauffe pendant 2 heures à reflux une solution de 2,1 g de l'acide préparé à l'exemple précédent dans 20 ml de chlorure de thionyle. On évapore sous vide, reprend le résidu par du toluène puis concentre. Le résidu huileux obtenu est utilisé tel quel à l'étape suivante.

B) 5-Ethoxy-1,3-dihydro-1-[2-méthoxy-4-[N-(2-méthylphényl)carbamoyl] benzyl]-3-spirocyclohexaneindol-2-one.

On laisse 24 heures sous agitation à TA une solution contenant 860 mg du chlorure d'acide préparé à l'étape précédente, 0,2 ml de 2-méthylaniline, 2 ml de TEA dans 80 ml de DCM. On effectue des lavages successifs par de l'eau, HCl 1N, de l'eau, une solution de $NaHCO_3$ saturée puis de l'eau. Après séchage sur sulfate de sodium et évaporation des solvants, le résidu est chromatographié sur silice en éluant par DCM/acétone (98/2, v/v). Le produit attendu cristallise dans MeOH, m = 0,082 g, F = 168°C.

EXEMPLE 8

5-Ethoxy-1,3-dihydro-1-(2-méthoxy-4-nitrobenzyl)-3-spirocyclohexaneindol-2-one.

A une solution de 4,9 g de 5-éthoxy-1,3-dihydro-3-spirocyclohexaneindol-2-one dans 40 ml de DMF, on ajoute par portions 0,66 g d'hydrure de sodium à 80 % dans l'huile. Après 15 minutes sous agitation, on introduit 5,4 g de 1-bromométhyl-2-méthoxy-4-nitrobenzène et on laisse 24 heures sous agitation à TA. Le solvant est évaporé sous vide puis on reprend par de l'eau, extrait au DCM, lave à l'eau et sèche sur sulfate de sodium. Après évaporation du solvant, on chromatographie sur silice en éluant par DCM/AcOEt (98/2, v/v). On obtient le produit attendu qui est repris par MeOH et essoré, m = 6,7 g, F = 170°C.

EXEMPLE 9

5-Ethoxy-1,3-dihydro-1-(2-méthoxy-4-aminobenzyl)-3-spirocyclohexaneindol-2-one.

On hydrogène à TA, pendant 24 heures, sous une pression de 50 bars un mélange de 6,157 g du composé préparé à l'exemple précédent et quelques grammes de Nickel de Raney® dans 270 ml d'éthanol à 95. On filtre le milieu réactionnel sur Célite®, lave au DCM puis évapore sous vide les solvants. Le produit attendu cristallise dans MeOH, m = 4,1 g, F = 81°C.

EXEMPLE 10

5-Ethoxy-1,3-dihydro-1-[4-(Δ3-pyrrolin-1-yl)-2-méthoxybenzyl]-3-spirocyclohexaneindol-2-one.

On mélange 1,52 g du composé préparé à l'exemple précédent, 1,05 g de cis 1,4-dichlorobut-2-ène et 1,74 ml de TEA dans 35 ml de DMF et on chauffe à reflux pendant 30 minutes. On évapore le solvant sous vide, reprend par de l'eau puis extrait par AcOEt, lave à l'eau et sèche sur sulfate de sodium. Après évaporation du solvant, on chromatographie sur silice en éluant par DCM/MeOH (98/2, v/v). Le composé attendu cristallise dans l'éther iso, m = 0,835 g, F = 114°C.

EXEMPLE 11

1-(4-Diméthylamino-2-méthoxybenzyl)-5-éthoxy-1,3-dihydro-3-spirocyclohexaneindol-2-one.

On mélange 420 mg du composé préparé à l'exemple 9 et 4 ml de formaldehyde à 37 % dans 10 ml d'acétonitrile ; on ajoute 0,5 g de cyanoborohydrure de sodium et 0,15 ml d'AcOH puis on laisse 48 heures sous agitation. Après avoir évaporé le solvant, on reprend par de l'eau puis extrait par AcOEt et sèche sur sulfate de sodium. On évapore le solvant puis le résidu est chromatographié sur silice en éluant par DCM/AcOEt (90/10, v/v). Le composé attendu cristallise dans l'heptane, m = 0,29 g, F = 138°C.

EXEMPLE 12

5-Ethoxy-1-[4-(2-éthylbutyroylamino)-2-méthoxybenzyl]-1,3-dihydro-3-spirocyclohexaneindol-2-one.

420 mg du composé préparé à l'exemple 9 et 1,65 ml de TEA sont placés dans 20 ml de DCM, on ajoute lentement 0,5 g de chlorure de 2-éthylbutyroyle et on laisse sous agitation pendant 2 heures à TA. On lave à l'eau la phase organique, sèche sur sulfate de sodium et évapore sous vide. Le résidu est chromatographié sur silice en éluant par DCM/MeOH (99/1, v/v). Le produit attendu cristallise dans un mélange pentane/éther iso, m = 0,30 g, F = 119°C.

EXEMPLE 13

1-(4-Diéthylaminoacétylamino-2-méthoxybenzyl)-5-éthoxy-1,3-dihydro-3-spirocyclohexaneindol-2-one.

A) 1-(4-Chloroacétylamino-2-méthoxybenzyl)-5-éthoxy-1,3-dihydro-3-spirocyclohexaneindol-2-one.

On prépare une solution de 420 mg du composé préparé à l'exemple 9 et 1 ml de TEA dans 12,5 ml de DCM, on ajoute 0,27 ml de chlorure de chloroacétyle et laisse 2 heures sous agitation. Après lavage à l'eau, on sèche sur sulfate de sodium, et évapore les solvants puis on effectue une chromatographie sur silice en éluant par DCM/MeOH (98/2, v/v). Le produit attendu cristallise dans l'éther iso, m = 0,36 g, F = 204°C.

B) 1-(4-Diéthylaminoacétylamino-2-méthoxybenzyl)-5-éthoxy-1,3-dihydro-3-spiro-cyclohexaneindol -2-one.

On laisse 48 heures sous agitation à TA, une solution contenant 340 mg du composé préparé à l'étape précédente et 7 ml de diéthylamine dans 15 ml de THF. On évapore le solvant sous vide puis on chromatographie le résidu sur silice en éluant par DCM/MeOH (98/2, v/v). Le produit attendu cristallise dans un mélange pentane/éther iso, m = 0,165 g, F = 75°C.

EXEMPLE 14

5-Ethoxy-1,3-dihydro-1-[2-méthoxy-4-(*ortho*-méthylbenzamido)benzyl]-3-spirocyclohexaneindol-2-one.

On laisse 24 heures sous agitation à TA une solution contenant 285 mg du composé préparé à l'exemple 9, 0,1 ml de chlorure d'*ortho*-méthylbenzoyle et 0,75 mg de TEA dans 30 ml de DCM. On lave à l'eau, sèche sur sulfate de sodium et évapore le solvant puis on chromatographie sur silice en éluant par DCM/MeOH (99,5/0,5, v/v). Le produit attendu cristallise dans l'éther iso, m = 285 mg, F = 158°C.

EXEMPLE 15

5-Chloro-1,3-dihydro-1-(2-méthoxy-4-nitrobenzyl)-3-spirocyclohexaneindol-2-one.

On ajoute par portions 0,528 g d'hydrure de sodium à 80 % dans l'huile à une solution de 4,434 g de 5-chloro-1,3-dihydro-3-spirocyclohexaneindol-2-one dans 40 ml de DMF. On refroidit par un bain de glace puis on ajoute lentement 5,4 g de 1-bromométhyl-2-méthoxy-4-nitrobenzène et on laisse 24 heures sous agitation à TA. On extrait à l'éther, lave à l'eau, sèche sur sulfate de sodium puis on concentre le solvant. On chromatographie sur silice en éluant au DCM. Le produit attendu cristallise dans le méthanol, m = 6,13 g, F = 180°C.

EXEMPLE 16

1-(4-Amino-2-méthoxybenzyl)-5-chloro-1,3-dihydro-3-spirocyclohexaneindol-2-one.

On hydrogène sous pression de 50 bars, à TA, pendant 24 heures, un mélange de 5,8 g du composé préparé à l'exemple précédent et quelques grammes de Nickel de Raney ® dans 250 ml d'éthanol à 95°. On filtre sur Célite ® le catalyseur puis on évapore le filtrat sous vide. Le produit attendu cristallise dans le méthanol, m = 3,25 g, F = 88°C.

EXEMPLE 17

5-Chloro-1,3-dihydro-1-(4-phénoxycarbonylamino-2-méthoxybenzyl)-3-spirocyclohexaneindol-2-one.

740 mg du composé préparé à l'exemple précédent sont placés dans 25 ml de THF, on ajoute 210 mg de soude dans 1,7 ml d'eau et on refroidit vers 5-10°C avant d'ajouter goutte à goutte 0,9 ml de chloroformiate de phényle dans 25 ml de THF. On laisse sous agitation 2 heures à TA. On évapore le solvant, reprend par de l'eau puis on extrait à l'éther et lave à l'eau. Après séchage sur sulfate de sodium et évaporation du solvant, on chromatographie sur silice en éluant par un mélange DCM/MeOH (98/2, v/v). Le produit attendu cristallise dans l'éthanol, m = 0,63 g, F = 196°C.

EXEMPLE 18

5-Chloro-1,3-dihydro-1-[2-méthoxy-4-(N'-méthyluréido)benzyl]-3-spirocyclohexaneindol-2-one.

On refroidit à 0°C une solution de 0,30 g du composé préparé à l'exemple 16 dans 20 ml de DCM et on ajoute 0,05 ml d'isocyanate de méthyle. Après 24 heures sous agitation à TA, on lave à l'eau, sèche sur sulfate de sodium et évapore sous vide. On chromatographie sur silice en éluant par DCM/MeOH (98/2, v/v). Le composé attendu cristallise dans l'éther iso, m = 0,280 g, F = 230°C.

EXEMPLE 19

5-Chloro-1,3-dihydro-1-[4-(3,4-diméthoxybenzènesulfonylamino)-2-méthoxybenzyl]-3-spirocyclohexane indol-2-one.

A une solution de 280 mg du composé préparé à l'exemple 16 dans 10 ml de pyridine, on ajoute 200 mg de chlorure de 3,4-diméthoxybenzènesulfonyle et on laisse 24 heures sous agitation à TA. On évapore le solvant sous vide puis reprend par de l'eau. On extrait au DCM, lave par de l'acide chlorhydrique 1N, puis par de l'eau, sèche sur sulfate de sodium et évapore sous vide. Le composé attendu cristallise dans l'éthanol, m = 0,31 g, F = 180°C.

EXEMPLE 20

5-Chloro-1,3-dihydro-1-(4-diméthylaminosulfonamido-2-méthoxybenzyl)-3-spirocyclohexaneindol-2-one.

On laisse 72 heures sous agitation, une solution contenant 371 mg du composé préparé à l'exemple 16 et 0,12 ml de chlorure de diméthylaminosulfamoyle dans 10 ml de pyridine. On évapore le solvant, reprend par de l'eau puis on extrait à l'éther, lave par de l'acide chlorhydrique 1N et par de l'eau. Après séchage sur sulfate de sodium et évaporation du solvant, on chromatographie sur silice en éluant par un mélange DCM/MeOH (99,4/0,6 ; v/v). Le produit attendu cristallise dans l'éther iso. m = 0,160 g, F = 164°C.

EXEMPLE 21

5-Chloro-1-[4-(N',N'-diéthyluréido)-2-méthoxybenzyl]-1,3-dihydro-3-spirocyclohexaneindol-2-one.

A une solution de 295 mg du composé préparé à l'exemple 17 dans 20 ml d'EtOH et 5,5 ml de DCM on ajoute 1,64 ml de diéthylamine et on laisse 24 heures sous agitation à TA. On évapore les solvants sous vide et on chromatographie sur silice en éluant par un mélange DCM/MeOH (99/1, v/v). Le produit attendu est repris par de l'heptane et essoré, m = 0,235 g, F = 108°C.

En utilisant les modes opératoires décrits ci-dessus, on a préparé les composés selon l'invention décrits dans le tableau 3 ci-après.

TABLEAU 3

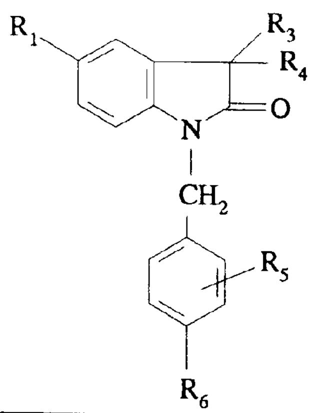

| N° Ex. | $R_1$ | $CR_3R_4$ | $R_5$ | $R_6$ | F°C ou RMN |
|---|---|---|---|---|---|
| 22 (a) | H | cyclohexane | MeO-3 | MeO- | 106 |
| 23 (b) | H | pipéridine-4 | MeO-2 | MeO- | RMN |
| 24 (c) | Cl- | cyclohexane | H- | MeO- | 80 |

| N° Ex. | $R_1$ | $CR_3R_4$ | $R_5$ | $R_6$ | F°C ou RMN |
|---|---|---|---|---|---|
| 25 (d) | Cl– | cyclohexane | MeO–2 | MeO– | 106 |
| 26 (d) | Cl– | 4,4–diméthyl–cyclohexane | MeO–2 | MeO– | 141 |
| 27 (a) | Cl– | cyclohexane | MeO–3 | MeO– | 117 |
| 28 (d) | MeO– | cyclohexane | MeO–2 | MeO– | 124 |
| 29 (a) | MeO– | cyclohexane | MeO–3 | MeO– | 110 |
| 30 (a) | EtO– | cyclohexane | MeO–3 | MeO– | 88 |
| 31 (e) | EtO– | cyclohexane | MeO–2 | tBuNHCO– | 187 |
| 32 (f) | Cl– | cyclohexane | H– | $F_3C$– | 90 |
| 33 (g) | H– | cyclohexane | H– | $O_2N$– | 118 |
| 34 (g) | Cl– | cyclohexane | H– | $O_2N$ | 139 |
| 35 (h) | Cl– | cyclohexane | Me–2 | $O_2N$– | 144 |

| N° Ex. | $R_1$ | $CR_3R_4$ | $R_5$ | $R_6$ | F°C ou RMN |
|---|---|---|---|---|---|
| 36 (i) | Cl- | cyclohexane | MeO-3 | $O_2N$- | 80 |
| 37 (g) | MeO- | cyclohexane | H- | $O_2N$- | 106 |
| 38 (h) | MeO- | cyclohexane | Me-2 | $O_2N$- | 133 |
| 39 (j) | MeO- | cyclohexane | MeO-2 | $O_2N$- | 200 |
| 40 (j) | EtO- | 4,4-diméthyl-cyclohexane | MeO-2 | $O_2N$- | 106 |
| 41 (k) | Cl- | cyclohexane | H- | $H_2N$- | 136 |
| 42 (k) | Cl- | cyclohexane | Me-2 | $H_2N$- | 157 |
| 43 (k) | Cl- | cyclohexane | MeO-3 | $H_2N$- | 113 |

| N° Ex. | $R_1$ | $CR_3R_4$ | $R_5$ | $R_6$ | F°C ou RMN |
|---|---|---|---|---|---|
| 44 (k) | MeO– | cyclohexane | H– | $H_2N$– | 192 |
| 45 (k) | MeO– | cyclohexane | MeO– | $H_2N$– | 165 |
| 46 (l) | Cl– | cyclohexane | MeO-2 | MeCONH– | 194 |
| 47 (l) | Cl– | cyclohexane | MeO-2 | $C_6H_{11}CONH$– | 143 |
| 48 (l) | Cl– | cyclohexane | MeO-2 | 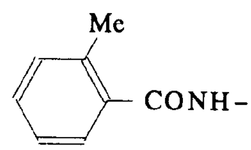 | 190 |
| 49 (l) | Cl– | cyclohexane | MeO-2 | 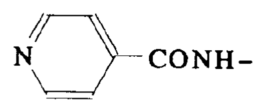 | 205 |
| 50 (l) | MeO– | cyclohexane | MeO-2 | MeCONH– | 202 |
| 51 (l) | MeO– | cyclohexane | H– | 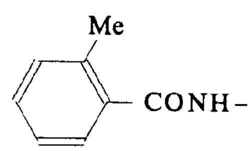 | 167 |
| 52 (l) | MeO– | cyclohexane | MeO-2 | 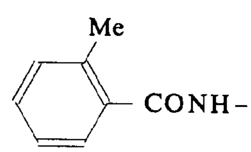 | 157 |
| 53 (m) | Cl– | cyclohexane | H– | PhOCONH– | 186 |

| N° Ex. | $R_1$ | $CR_3R_4$ | $R_5$ | $R_6$ | F°C ou RMN |
|---|---|---|---|---|---|
| 54 (m) | Cl– | cyclohexane | MeO–3 | PhOCONH– | 112 |
| 55 (n) | Cl– | cyclohexane | H– | $Et_2NCONH$– | 190 |
| 56 (n) | Cl– | cyclohexane | MeO–3 | $Et_2NCONH$– | 108 |
| 57 (o) | Cl– | cyclohexane | Me–2 | MeNHCONH– | 153 |
| 58 (n) | EtO– | cyclohexane | MeO–2 | $Et_2NCONH$– | 131 |
| 59 (p) | EtO– | cyclohexane | MeO–2 | 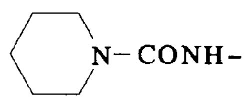 | 133 |
| 60 (d) | Cl– | N–méthyl–pipéridine–4 | MeO–2 | MeO– | 64 |
| 61 (m) | EtO– | cyclohexane | MeO–2 | PhOCONH– | 170 |
| 62 (j) | EtO– | 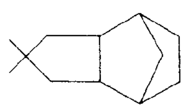 | MeO–2 | $O_2N$– | 88 |
| 63 (q) | EtO– | 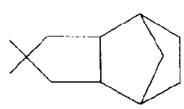 | MeO–2 | $H_2N$– | 96 |
| 64 (r) | EtO– | 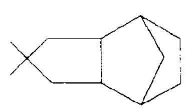 | MeO–2 | $Et_2NCONH$– | 126 |

| N° Ex. | $R_1$ | $CR_3R_4$ | $R_5$ | $R_6$ | F°C ou RMN |
|---|---|---|---|---|---|
| 65 (j) | EtO– | tétrahydropyrane -4 | MeO–2 | $O_2N$– | 163 |
| 66 (s) | EtO– | tétrahydropyrane -4 | MeO–2 | $H_2N$– | 171 |
| 67 (r) | EtO– | tétrahydropyrane -4 | MeO–2 | $Et_2NCONH$– | 98 |
| 68 (1) (j) | EtO– | 4–méthoxycyclo–hexane | MeO–2 | $O_2N$– | huile |
| 69 (2) (s) | EtO– | 4–méthoxycyclo–hexane | MeO–2 | $H_2N$– | 92 |
| 70 (3) (r) | EtO– | 4–méthoxycyclo–hexane | MeO–2 | $Et_2NCONH$– | 62 |

Spectre RMN à 200 MHz dans le DMSO de l'exemple 23

1,6–1,9 ppm : m : 4 H

2,9–3,3 ppm : m : 4 H

3,8 ppm : s : 3 H

3,9 ppm : s : 3 H

4,8 ppm : s : 2 H

6,5 ppm : d de d : 1 H

6,65 ppm : d : 1 H

6,85 ppm : d : 1 H

6,95 ppm : d : 1 H

7,1 ppm : t : 1 H

7,25 ppm : t : 1 H

7,6 ppm : d : 1 H

(a) On prépare ce composé selon le mode opératoire décrit à l'EXEMPLE 1.

(b) On prépare ce composé selon le mode opératoire suivant : on chauffe à reflux pendant 4 heures un mélange de 0,79 g du composé obtenu à l'EXEMPLE 3, 1,4 g de

KOH dans 5 ml d'EtOH 95. Après refroidissement, on ajoute de l'eau, extrait à l'éther, extrait la phase organique par une solution d'HCl 3N, basifie la phase aqueuse acide par ajout d'une solution à 10 % de NaOH, extrait à l'éther, lave à l'eau, sèche sur sulfate de sodium et évapore sous vide le solvant. On chromatographie le résidu sur silice en éluant par le mélange DCM/MeOH (80/20 ; v/v) puis (50/50 ; v/v). On obtient 0,31 g du produit attendu.

(c) On prépare ce composé selon le mode opératoire décrit à l'EXEMPLE 1 en utilisant le 1-chlorométhyl-4-méthoxybenzène.

(d) On prépare ce composé selon le mode opératoire décrit à l'EXEMPLE 2.

(e) On prépare ce composé selon le mode opératoire suivant : on laisse 1 heure sous agitation à TA, un mélange de 0,6 g du composé obtenu à l'EXEMPLE 6, 1 ml de *tert*-butylamine, 0,73 g de BOP, 2 ml de DIPEA et 45 ml de DCM. On lave par une solution d'HCl 1N, à l'eau, par une solution saturée de $NaHCO_3$, à l'eau, sèche sur $Na_2SO_4$ et évapore sous vide le solvant. On chromatographie le résidu sur silice en éluant par le mélange DCM/MeOH (98/2 ; v/v). On obtient 0,295 g du produit attendu après cristallisation dans l'éther iso.

(f) On prépare ce composé selon le mode opératoire décrit à l'EXEMPLE 1 à partir du 1-bromométhyl-4-trifluorométhylbenzène.

(g) On prépare ce composé selon le mode opératoire décrit à l'EXEMPLE 8 à partir du 1-bromométhyl-4-nitrobenzène.

(h) On prépare ce composé selon le mode opératoire décrit à l'EXEMPLE 8 à partir du 1-bromométhyl-2-méthyl-4-nitrobenzène.

(i) On prépare ce composé selon le mode opératoire décrit à l'EXEMPLE 8 à partir du 1-bromométhyl-3-méthoxy-4-nitrobenzène.

(j) On prépare ce composé selon le mode opératoire décrit à l'EXEMPLE 8.

(k) On prépare ce composé selon le mode opératoire décrit à l'EXEMPLE 9 à partir du composé nitré correspondant.

(l) On prépare ce composé selon le mode opératoire décrit à l'EXEMPLE 12 à partir du composé aminé correspondant et en utilisant les chlorures d'acides appropriés.

(m) On prépare ce composé selon le mode opératoire décrit à l'EXEMPLE 17 à partir du composé aminé correspondant.

(n) On prépare ce composé selon le mode opératoire décrit à l'EXEMPLE 21 à partir du composé phénoxycarbonylamino correspondant.

(o) On prépare ce composé selon le mode opératoire décrit à l'EXEMPLE 18 à partir du composé aminé correspondant.

(p) On prépare ce composé selon le mode opératoire décrit à l'EXEMPLE 21 à partir du composé phénoxycarbonylamino correspondant, en utilisant la pipéridine.

(q) On prépare ce composé selon le mode opératoire suivant : On refroidit à 10°C un mélange de 1,26 g du composé de l'EXEMPLE 62, 1,37 g d'étain en poudre dans 15 ml d'EtOH, ajoute 2,73 ml d'HCl concentré, puis chauffe à 40°C pendant 1 heure. Après refroidissement, on ajoute une solution saturée de $NaHCO_3$, extrait à l'AcOEt, sèche sur $Na_2SO_4$ et évapore sous vide le solvant. On chromatographie le résidu sur silice en éluant au DCM. On obtient 0,93 g du produit attendu.

(r) On prépare ce composé selon les modes opératoires décrits à l'exemple 17 puis à l'exemple 21.

(s) On prépare ce composé selon le mode opératoire décrit au point (q) ci–dessus.

(1) On prépare ce composé à partir du composé obtenu à la préparation 14 (isomère B : composé le plus polaire).

(2) On prépare ce composé à partir du composé de l'exemple 68.

(3) On prépare ce composé à partir du composé de l'exemple 69.

## Revendications

1. Composé de formule :

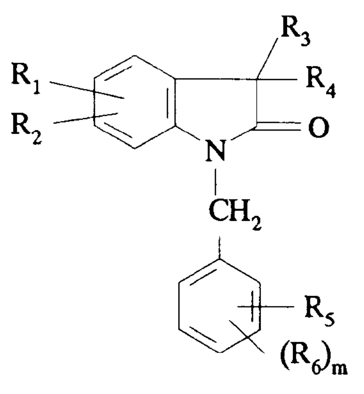

(I)

dans laquelle

- $R_1$ et $R_2$ représentent chacun indépendamment un hydrogène ; un halogéno ; un hydroxy ; un ω-halogéno($C_1$-$C_7$)alcoxy ; un ($C_1$-$C_7$)alkyle ; un trifluorométhyle ; un ($C_1$-$C_7$)alcoxy ; un polyhalogéno($C_1$-$C_7$)alcoxy ; un ω-hydroxy($C_2$-$C_7$)alcoxy, un ω-méthoxy($C_2$-$C_7$)alcoxy ; un ω-amino($C_2$-$C_7$)alcoxy libre ou substitué par un ou deux ($C_1$-$C_7$)alkyles ; un ($C_3$-$C_7$)cycloalkyloxy ; un ($C_3$-$C_7$)cycloalkylméthoxy ; un phénoxy ; un benzyloxy ; un ($C_1$-$C_7$)alkylthio ; un phénylthio ; un nitro ; un amino libre ou substitué par un ou deux ($C_1$-$C_7$)alkyles ; un cyano ; un formyle ; un ($C_1$-$C_7$)alkylcarbonyle ; un benzoyle ; un formyloxy ; un ($C_1$-$C_7$)alkylcarbonyloxy ; un benzoyloxy ; un ($C_1$-$C_7$)alkylsulfonamido ; un phénylsulfonamido ; un benzylsulfonamido ; un ($C_1$-$C_7$)alkylcarbonylamino ; un ($C_1$-$C_7$)alcoxycarbonylamino ; un uréido non substitué ou substitué par un phényle, un benzyle ou par un ou deux ($C_1$-$C_7$)alkyles ; un thiouréido non substitué ou substitué par un phényle, un benzyle ou par un ou deux ($C_1$-$C_7$)alkyles ; à la condition que $R_1$ et $R_2$ ne soient pas simultanément l'hydrogène ;
- $R_3$ et $R_4$ ensemble constituent un groupe $-(CH_2)_pX(CH_2)_q-$ ;

  ou
- -$R_3$ et $R_4$ ensemble avec le carbone auquel ils sont liés constituent un cycle hydrocarboné en $C_3$-

$C_{12}$, saturé ou insaturé, éventuellement condensé, non substitué ou substitué par un ou plusieurs groupes $(C_1-C_7)$alkyles, par un spirocycloalkyle en $C_3-C_5$, par un groupe oxo, par un ou deux hydroxy libres ou substitués par un groupe choisi parmi les groupes : $(C_1-C_4)$alkyle, $(C_1-C_2)$alcoxy$(C_1-C_4)$alkyle, phényl$(C_1-C_2)$alcoxy$(C_1-C_4)$alkyle, tétrahydrofuranyle et tétrahydropyranyle ;

- $R_5$ représente l'hydrogène ou l'une des valeurs désignées pour $R_6$ ;
- $R_6$ représente un halogène ; un $(C_1-C_7)$alkyle ; un trifluorométhyle ; un cyano ; un nitro ; un hydroxylamino ; un hydroxy ; un carboxy ; un guanidino non substitué ou substitué en position 1 par un $(C_1-C_7)$alkyle et/ou en position 3 par un ou deux $(C_1-C_7)$alkyles, un phényle ou un benzyle et/ou en position 2 par un cyano ; un groupe $OR_7$ ; un groupe $SR_7$ ; un formyle ; un $(C_1-C_7)$alkylcarbonyle ; un benzoyle ; un $(C_1-C_7)$alcoxycarbonyle ; un phénoxycarbonyle ; un benzyloxycarbonyle ; un groupe $-CONR_{17}R_{18}$ ; un groupe $-CSNR_{11}R_{19}$ ;un groupe $-SO_2NR_{20}R_{21}$ ; un $(C_1-C_7)$alkylsulfonamido ; un groupe $-NHSO_2-Ar$ ; un benzylsulfonamido ; un aminosulfonamido dans lequel l'amino est libre ou substitué par $R_{16}$ et $R_{22}$ ; un groupe $-NR_8R_9$ ; un groupe $-CO-NH-CR_{10}R_{23}-COR_{12}$ ; un groupe $-CH_2NR_8R_9$ ;
- $R_7$ représente un $(C_1-C_7)$alkyle ; un phényle ; un benzyle ; un $(C_3-C_7)$cycloalkyle ; un $(C_2-C_7)$alcényle ; un ω-halogéno$(C_2-C_7)$alkyle ; un polyhalogéno$(C_1-C_7)$alkyle ; un ω-hydroxy$(C_2-C_7)$alkyle ; un formyle ; un $(C_1-C_7)$alkylcarbonyle ; un benzoyle ; un ω-carboxy$(C_1-C_7)$alkyle ; un ω-$(C_1-C_7)$alcoxycarbonyl$(C_1-C_7)$alkyle ; un ω-benzyloxycarbonyl$(C_1-C_7)$alkyle ; un ω-amino$(C_2-C_7)$alkyle dans lequel le groupe amino est libre ou substitué par un ou deux $(C_1-C_7)$alkyles ou sous forme d'ion ammonium ; un ω-carbamoyl$(C_1-C_7)$alkyle dans lequel le carbamoyle est libre ou substitué par un ou deux $(C_1-C_7)$alkyles ;
- $R_8$ et $R_9$ représentent chacun indépendamment un hydrogène ; un $(C_1-C_7)$alkyle ; un groupe $-CH_2-Ar$ ; $R_9$ peut de plus représenter un groupe -Ar ; un $(C_3-C_8)$alcényle ; un formyle ; un $(C_1-C_7)$alkylcarbonyle ; un $(C_1-C_7)$alkylthiocarbonyle ; un $(C_3-C_7)$cycloalkylcarbonyle ; un $(C_3-C_7)$cycloalkylthiocarbonyle ; un ω-amino$(C_2-C_7)$alkylcarbonyle dans lequel l'amino est libre ou substitué par un ou deux $(C_1-C_7)$alkyles ; un ω-hydroxy$(C_1-C_7)$alkylcarbonyle ; un ω-benzyloxy$(C_1-C_7)$alkylcarbonyle ; un pyridylcarbonyle ; un méthylpyridylcarbonyle ; un thiénylcarbonyle ; un groupe -CO-Ar ; un groupe-CO-$CH_2$-Ar ; un $(C_1-C_7)$alcoxycarbonyle ; un phénoxycarbonyle ; un phénoxythiocarbonyle ; un benzyloxycarbonyle ; un groupe $-CO-CR_{10}R_{23}-NR_{11}R_{19}$, un groupe $-CR_{10}R_{23}COR_{12}$, un groupe $-(CH_2)_tCOR_{12}$ ; un groupe $-CO(CH_2)_uCOR_{12}$ ; un groupe $-CONR_{14}R_{24}$ ; un groupe $-CSNR_{14}R_{24}$ ; un radical hétérocyclique choisi parmi pyrazolyle, imidazolyle, triazolyle, tétrazolyle, pyridyle, pyridazinyle, pyrimidinyle, thiazolyle ;
- ou bien $R_8$ et $R_9$ ensemble avec l'atome d'azote auquel ils sont liés constituent l'hydantoïne, la N-méthylhydantoïne, ou un radical hétérocyclique choisi parmi le pyrrol-1-yle, le Δ3-pyrrolin-1-yle, le pyrrolidin-1-yle, l'isoindolin-2-yle dans lequel le noyau benzénique est non substitué ou substitué par un halogène, un $(C_1-C_7)$alkyle, un trifluorométhyle ou un méthoxy ;
- $R_{10}$ et $R_{23}$ représentent chacun indépendamment l'hydrogène ; un $(C_1-C_7)$alkyle ; un benzyle ; ou $R_{10}$ et $R_{23}$ ensemble avec l'atome de carbone auquel ils sont liés constituent un $(C_3-C_7)$cycloalkyle ;
- $R_{11}$ et $R_{19}$ représentent chacun indépendamment l'hydrogène ou un $(C_1-C_7)$alkyle ;
- $R_{12}$ représente un hydroxy ; un $(C_1-C_7)$alcoxy ; un amino libre ou substitué par un ou deux $(C_1-C_7)$alkyles ;
- $R_{13}$ représente l'hydrogène ; un $(C_1-C_7)$alkyle ; un phényle ; un benzyle ; un formyle ; un $(C_1-C_7)$alkylcarbonyle ; un benzoyle ; un $(C_1-C_7)$alcoxycarbonyle ; un carbamoyle non substitué ou substitué par un ou deux $(C_1-C_7)$alkyles ;
- $R_{14}$ et $R_{24}$ représentent chacun indépendamment l'hydrogène ; un $(C_1-C_7)$alkyle ; $R_{24}$ peut de plus représenter un $(C_1-C_7)$alkyle substitué par $R_{15}$ ; un groupe -Ar ; un $(C_3-C_7)$cycloalkyle ; un adamantyle ;
- ou $R_{14}$ et $R_{24}$ ensemble avec l'atome d'azote auquel ils sont liés constituent un hétérocycle choisi parmi : la morpholine, la thiomorpholine, la pipérazine, l'azétidine, la pyrrolidine, la pipéridine ou la perhydroazépine, ledit hétérocycle étant non substitué ou substitué par un ou plusieurs groupes méthyles, par un phényle, par un groupe amino libre ou portant un groupe protecteur ;
- $R_{15}$ représente un groupe Ar ; un pyridyle ; un hydroxy ; un $(C_1-C_7)$alcoxy ; un groupe $-NR_{11}R_{19}$ ; un carboxy ; un $(C_1-C_7)$alcoxycarbonyle ;
- $R_{16}$ et $R_{22}$ représentent chacun indépendamment un hydrogène ou un $(C_1-C_7)$alkyle ; ou $R_{16}$ et $R_{22}$ ensemble avec l'atome d'azote auquel ils sont liés constituent un hétérocycle choisi parmi : l'azétidine, la pyrrolidine, la pipéridine, la pipérazine ou la perhydroazépine, le dit hétérocycle étant non substitué ou substitué par un ou plusieurs groupes méthyles ;
- $R_{17}$ et $R_{18}$ représentent chacun indépendamment l'hydrogène ou un $(C_1-C_8)$alkyle ; $R_{18}$ peut de plus représenter un $(C_3-C_7)$cycloalkyle non substitué ou substitué par un $(C_1-C_4)$alkyle ; un groupe -Ar ;

un pyridyle ; un méthylpyridyle ; un pipérid-4-yle substitué en position 1 par $R_{27}$ ; un pipérid-1-yle ; un pyrrolidin-1-yle ; un morpholin-4-yle ; un $(C_1-C_7)$alkyle substitué par un ou plusieurs halogènes ou par $R_{26}$.

- ou bien $R_{17}$ et $R_{18}$ ensemble avec l'atome d'azote auquel ils sont liés constituent un radical hétérocyclique $R_{25}$ ;
- $R_{20}$ et $R_{21}$ représentent chacun indépendamment l'hydrogène ou un $(C_1-C_7)$alkyle ;
- ou bien $R_{20}$ et $R_{21}$ ensemble avec l'atome d'azote auquel ils sont liés constituent un radical hétérocyclique $R_{25}$ ;
- $R_{25}$ représente un morpholin-4-yle ; un thiomorpholine-4-yle ; un azétidin-1-yle non substitué ou substitué en position 3 par un groupe $-NR_{11}R_{19}$, un $(C_1-C_7)$alkyle, un phényle, un benzyle ou un $(C_1-C_7)$alkylcarbonyle ; un perhydroazépin-1-yle ; un pipérazin-1-yle non substitué ou substitué en position 4 par un $(C_1-C_7)$alkyle, un phényle, un benzyle, un $(C_1-C_7)$alkylcarbonyle, un $(C_1-C_7)$alcoxycarbonyle ou un benzyloxycarbonyle ; un pipérid-1-yle non substitué ou substitué en position 4 par un $(C_1-C_7)$alkyle, un phényle, un benzyle, un $(C_1-C_7)$alkylcarbonyle, un groupe $-NR_{11}R_{19}$ ; un pyrrolidin-1-yle non substitué ou substitué par un $(C_1-C_7)$alkyle, un phényle, un benzyle, un $(C_1-C_7)$alkylcarbonyle, un hydroxyméthyle, un carboxy, un $(C_1-C_7)$alcoxycarbonyle, un carbamoyle non substitué ou substitué par un ou deux $(C_1-C_7)$alkyles ;
- $R_{26}$ représente un hydroxy ; un $(C_1-C_7)$alcoxy ; un cyano ; un carboxy ; un $(C_1-C_7)$alcoxycarbonyle ; un benzyloxycarbonyle ; un groupe $-NR_{11}R_{19}$ ; un carbamoyle libre ou substitué par un ou deux $(C_1-C_7)$alkyles ; un pyrrolidin-1-ylcarbonyle ; un pipérid-1-ylcarbonyle ; un perhydroazépin-1-ylcarbonyle ; un groupe -Ar ; un $(C_3-C_7)$cycloalkyle ; un adamantyle ; un radical hétérocyclique choisi parmi un pyridyle, un méthylpyridyle, un furanyle, un tétrahydrofuranyle, un thiényle, un méthylthiényle, un pyrrolidin-1-yle, un pipérid-1-yle, un perhydroazépin-1-yle ;
- $R_{27}$ représente un hydrogène ; un $(C_1-C_7)$alkyle ; un phényle ; un benzyle ; un formyle ; un $(C_1-C_7)$alkylcarbonyle ; un benzoyle ; un $(C_1-C_7)$alcoxycarbonyle ; un phénoxycarbonyle ; un carbamoyle libre ou substitué par un ou deux $(C_1-C_7)$alkyles.
- Ar représente un phényle non substitué ou substitué une ou plusieurs fois par un substituant choisi parmi : un atome d'halogène, un $(C_1-C_7)$alkyle, un trifluorométhyle, un hydroxy, un $(C_1-C_7)$alcoxy, un carboxy, un $(C_1-C_7)$alcoxycarbonyle, un $(C_1-C_7)$alkylcarbonyloxy, un nitro, un cyano, un amino, un $(C_1-C_7)$alkylamino, un $(C_1-C_7)$dialkylamino, lesdits substituants étant identiques ou différents ;
- m est 1 ou, lorsque $R_6$ est un halogène, un $(C_1-C_7)$alkyle ou un $(C_1-C_7)$alcoxy, m peut également être 2, 3 ou 4 ou bien $(R_6)_m$ peut représenter m substituants ayant différentes valeurs choisies parmi halogène, $(C_1-C_7)$alkyle ou $(C_1-C_7)$alcoxy ;
- p et q représentent chacun un nombre entier, leur somme peut varier de 3 à 6 ;
- t représente un nombre entier qui peut varier de 2 à 5 ;
- u représente un nombre entier qui peut varier de 0 à 3 ;
- X représente l'oxygène, un groupe $NR_{13}$, un groupe $N(O)R_{13}$, un groupe $S(O)_X$ ;
- x représente 0, 1 ou 2 ;

ainsi que leurs sels éventuels.

2. Composé selon la revendication 1, caractérisé en ce que $R_1$ est en position 5 du 1,3-dihydro-indol-2-one et $R_2$ est l'hydrogène.

3. Composé selon l'une des revendications 1 ou 2, caractérisé en ce que $R_1$ est un atome de chlore ou de fluor ou un groupe éthoxy en position 5 du 1,3-dihydroindol-2-one et $R_2$ est l'hydrogène.

4. Composé selon l'une quelconque des revendications 1 à 3, caractérisé en ce que $R_3$ et $R_4$ ensemble avec le carbone auquel ils sont liés constituent un cycle hydrocarboné en $C_3-C_{12}$.

5. Composé selon l'une quelconque des revendications 1 à 3, caractérisé en ce que $R_3$ et $R_4$ ensemble avec le carbone auquel ils sont liés constituent un cycloheptane, un adamantane, un tricyclo $[5.2.1.0^{2,6}]$décane, un tricyclo $[5.2.1.0^{2,6}]$dec-8-ène, un bicyclo [2.2.1]heptane, un bicyclo [3.3.1]nonane, ou un cyclohexane non substitué ou substitué par un spirocycloalkyle en $C_3-C_5$, par un ou deux alkyles en $C_1-C_7$ ou par un alcoxy en $C_1-C_4$.

6. Composé selon l'une quelconque des revendications 1 à 3, caractérisé en ce que $R_3$ et $R_4$ ensemble avec le carbone auquel ils sont liés constituent un cycle pipéridine-4 non substitué ou substitué sur l'azote par un alkyle en $C_1-C_7$.

7. Composé selon l'une quelconque des revendications 1 à 6, caractérisé en ce que $R_5$ représente soit l'hydrogène soit un groupe méthoxy en position 2 et $R_5$ en position 4 représente un groupe choisi parmi :
   - un méthoxy ;
   - un ($C_1$-$C_7$)alkylcarboxamido ;
   - un groupe -NHCO-Ar ;
   - un groupe -$CONR_{17}R_{18}$ ;
   - un groupe $NR_5CONR_{14}R_{24}$ ;

   dans lesquels Ar, $R_{17}$, $R_{18}$, $R_8$, $R_{14}$ et $R_{24}$ sont tels que définis pour (I) dans la revendication 1.

8. Procédé pour l'obtention d'un composé de formule (I) tel que défini dans l'une quelconque des revendications 1 à 7, caractérisé en ce que :

   a) on fait agir sur un 1,3-dihydro-indol-2-one disubstitué en position 3, de formule :

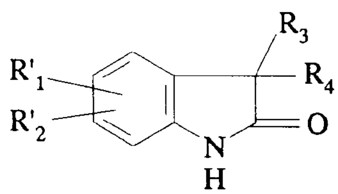

(II)

dans laquelle $R'_1$ et $R'_2$ représentent, respectivement, soit $R_1$ et $R_2$ tels que définis pour (I), dans la revendication 1 soit des groupes précurseurs de $R_1$, $R_2$ , et $R_3$, $R_4$ sont tels que définis pour (I) dans la revendication 1, un halogénure de benzyle de formule :

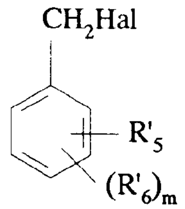

(III)

dans laquelle Hal représente un atome d'halogène, et $R'_5$ et $R'_6$ représentent, respectivement, soit $R_5$ et $R_5$ tels que définis ci-dessus pour (I) dans la revendication 1, soit des groupes précurseurs de $R_5$ et $R_6$ ; et,

b) soit, lorsque $R'_1 = R_1$, $R'_2 = R_2$, $R'_5 = R_5$ et $R'_6 = R_6$, on isole le composé de formule (I) ainsi obtenu ;

c) soit, lorsque l'un quelconque des groupes $R'_1$, $R'_2$, $R'_5$ et/ou $R'_6$ représente respectivement un groupe précurseur de $R_1$, $R_2$, $R_5$ et/ou $R_6$, on soumet le composé obtenu à l'étape a, à un traitement ultérieur pour préparer le composé de formule (I) par transformation de l'un quelconque des groupes $R'_1$, $R'_2$, $R'_5$ et/ou $R'_6$ en, respectivement, $R_1$, $R_2$, $R_5$ et/ou $R_6$.

9. Composition pharmaceutique contenant à titre de principe actif un composé selon l'une quelconque des revendications 1 à 7.

10. Composition pharmaceutique contenant un composé selon l'une quelconque des revendications 1 à 7 en association avec un autre principe actif.

11. Composition pharmaceutique contenant deux composés selon l'une quelconque des revendications 1 à 7, l'un étant un antagoniste spécifique du récepteur $V_1$ et l'autre étant un antagoniste spécifique du récepteur $V_2$.

12. Composition pharmaceutique contenant deux composés selon l'une quelconque des revendications 1 à 7, l'un étant un antagoniste spécifique du récepteur $V_1$ et l'autre étant un antagoniste spécifique de l'ocytocine.

Office européen des brevets

# RAPPORT DE RECHERCHE EUROPEENNE

Numero de la demande
EP 94 40 1738

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| Y,D | US-A-4 226 860 (DEMERSON ET AL.)<br>* abrégé; exemple 3 * | 1,9 | C07D209/96<br>C07D471/10<br>C07D491/10<br>//(C07D471/10, 221:00, 209:00), (C07D491/10, 311:00,209:00) |
| Y,D | EP-A-0 344 634 (BOEHRINGER MANNHEIM GMBH)<br>* page 3; revendication 1 * | 1,9 | |
| A,D | EP-A-0 028 906 (IMPERIAL CHEMICAL IND., LTD.)<br>* page 13; revendication 1 * | 1,9 | |
| A | CHEMICAL ABSTRACTS, vol. 73, no. 1, 6 Juillet 1970, Columbus, Ohio, US; abstract no. 3739r,<br>* abrégé *<br>& ARCH. PHARM., vol.303, no.2, 1970 pages 183 - 191<br>H. SCHAEFER | 1,9 | |
| A | EP-A-0 065 407 (IMPERIAL CHEMICAL IND., LTD.)<br>* page 22; revendication 1 * | 1,9 | **DOMAINES TECHNIQUES RECHERCHES (Int.Cl.6)** |
| A | EP-A-0 079 675 (PFIZER INC.)<br>* page 10; revendication 1 * | 1,9 | C07D<br>A61K |
| A,P,D | WO-A-93 15051 (ELF SANOFI)<br>* le document en entier * | 1,9 | |
| A,D | EP-A-0 469 984 (SANOFI)<br>* abrégé; page 10 * | 1,9 | |
| A | EP-A-0 450 761 (MERCK & CO., INC.)<br>* pages 24-25; revendication 1 * | 1,9 | |

-/--

Le présent rapport a été établi pour toutes les revendications

| Lieu de la recherche | Date d'achèvement de la recherche | Examinateur |
|---|---|---|
| BERLIN | 10 Novembre 1994 | Frelon, D |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numero de la demande
EP 94 40 1738

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A,D | PATENT ABSTRACTS OF JAPAN<br>vol. 15, no. 332 (C-0861) 23 Août 1991<br>& JP-A-03 127 732 (OTSUKA PHARM. CO., LTD.) 30 Mai 1991<br>* abrégé *<br>--- | 1,9 | |
| A,D | US-A-4 803 217 (R.E. SCHWARTZ ET AL.)<br>* abrégé *<br>----- | 1,9 | |

DOMAINES TECHNIQUES RECHERCHES (Int.Cl.6)

Le présent rapport a été établi pour toutes les revendications

| Lieu de la recherche | Date d'achèvement de la recherche | Examinateur |
|---|---|---|
| BERLIN | 10 Novembre 1994 | Frelon, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..................................................................
& : membre de la même famille, document correspondant

EPO FORM 1503 03.82 (P04C02)